# EUROPÄISCHE PATENTSCHRIFT

(11) **EP 1 226 155 B1**
(45) Veröffentlichungstag und Bekanntmachung des Hinweises auf die Patenterteilung: **02.05.2007**
(21) Anmeldenummer: 00981233.0
(22) Anmeldetag: 02.11.2000
(51) Int. Cl.: C07J 1/00, A61K 31/565

(54) **18-NOR-STEROIDE ALS SELEKTIV WIRKSAME ESTROGENE**
18-NOR-STEROIDS AS SELECTIVELY ACTIVE ESTROGENS
18-NOR-STEROIDES UTILISES COMME OESTROGENES A EFFET SELECTIF

(30) Priorität: 02.11.1999 DE 19954105; 10.03.2000 US 188197 P
(43) Veröffentlichungstag der Anmeldung: 31.07.2002
(73) Patentinhaber: Bayer Schering Pharma AG, 13353 Berlin (DE)
(72) Erfinder: HILLISCH, Alexander, 07743 Jena (DE); BOIDOL, Werner, 14197 Berlin (DE); SCHWEDE, Wolfgang, 13467 Berlin (DE); ESPERLING, Peter, 12107 Berlin (DE); SAUER, Gerhard, 13465 Berlin (DE); HEGELE-HARTUNG, Christa, 45470 Mülheim/Ruhr (DE); KOLLENKIRCHEN, Uwe, 12209 Berlin (DE); FRITZEMEIER, Karl-Heinrich, 13505 Berlin (DE)
(86) Internationale Anmeldenummer: PCT/EP2000/010804
(87) Internationale Veröffentlichungsnummer: WO 2001/032680

(56) Entgegenhaltungen:
- WO-A-00/63228
- GB-A- 1 298 974
- US-A- 3 016 389
- US-A- 3 407 219
- US-A- 3 574 197
- US-A- 3 576 829
- US-A- 3 755 384
- US-A- 4 605 649
- US-A- 4 705 783
- CHEMICAL ABSTRACTS, vol. 63, no. 5, 30. August 1965 (1965-08-30) Columbus, Ohio, US; abstract no. 5705c, L. N. IVANOVA ET AL: "Some chemical transformations or methyl ether of cis-18-nor-delta-9(11)-estra-15,17-dione" XP002166902 -& DATABASE CROSSFIRE BEILSTEIN [Online] BEILSTEIN INSTITUT ZUR FÖRDERUNG DER CHEMISCHEN WISSENSCHAFTEN, FRANKFURT AM MAIN, DE; Database accession no. 2625354 XP002166904 & IZV. AKAD. NAUK SSSR, SER. KHIM., Nr. 5, 1965, Seiten 843-846,
- W. F. JOHNS: "Retropinacol Rearrangement of Estradiol 3-Methyl Ether" JOURNAL OF ORGANIC CHEMISTRY., Bd. 26, 1961, Seiten 4583-4591, XP002166896 AMERICAN CHEMICAL SOCIETY. EASTON., US ISSN: 0022-3263
- CHEMICAL ABSTRACTS, vol. 55, no. 6, 20. März 1961 (1961-03-20) Columbus, Ohio, US; abstract no. 5754f, R. A. EDGREN ET AL: "Estrogenic Effects of 18-nor-17.beta.estradiol and 18-nor-estrone" XP002166903 & PROCEEDINGS OF THE SOCIETY FOR EXPERIMENTAL BIOLOGY & MEDICINE., Bd. 105, 1960, Seiten 286-287, ACADEMIC PRESS INC. NEW YORK., US ISSN: 0037-9727
- W. F. JOHNS: "Synthesis of 18,19-Dinor Steroids" JOURNAL OF THE AMERICAN CHEMICAL SOCIETY., Bd. 80, Nr. 23, 17. Dezember 1958 (1958-12-17), Seiten 6456-6457, XP002166897 AMERICAN CHEMICAL SOCIETY, WASHINGTON, DC., US ISSN: 0002-7863
- DATABASE CHEMABS [Online] CHEMICAL ABSTRACTS SERVICE, COLUMBUS, OHIO, US; KANAZAWA, GIICHI: "Clinical effect of a contraceptive, Sophia 32" retrieved from STN Database accession no. 72:18893 XP002166905 & HORUMON TO RINSHO (1969), 17(7), 611-14 ,
- HAEHNEL, ROLAND ET AL: "Specificity of the estrogen receptor of human uterus" J. STEROID BIOCHEM. (1973), 4(1), 21-31 , XP002166898
- HAEHNEL, ROLAND ET AL: "Steroid specificity of the estrogen receptor of human breast carcinoma" J. STEROID BIOCHEM. (1974), 5(2), 119-22 , XP002166899
- KUHL, ALEXANDER ET AL: "17.alpha.-(4-Chlorobenzoyloxy)-3-methoxy- 13.alpha.-gona-1,3,5(10)- triene" ACTA CRYSTALLOGR., SECT. C: CRYST. STRUCT. COMMUN. (1998), C54(4), 521-523 , XP002166900
- M. M. COOMBS: "Potentially Carinogenic Cyclopenta[a]phenanthrenes(1,2-cyclopenten ophenanthranes). Part I. A New Synthesis of 15,16-Dihydro-17-oxo-cyclopenta[a]phenanth rene and the Phenanthrene Analogue of 18-Norestrone Methyl Ether" JOURNAL OF THE CHEMICAL SOCIETY, SECTION C: ORGANIC CHEMISTRY., Nr. 10, 1966, Seiten 955-962, XP002166901 CHEMICAL SOCIETY. LETCHWORTH., GB ISSN: 1472-7781
- DATABASE BIOSIS [Online] BIOSCIENCES INFORMATION SERVICE, PHILADELPHIA, PA, US; 1995 LI JONATHAN J ET AL: "Carcinogenic activities of various steroidal and nonsteroidal estrogens in the hamster kidney: Relation to hormonal activity and cell proliferation." Database accession no. PREV199598550304 XP002166906 & CANCER RESEARCH, Bd. 55, Nr. 19, 1995, Seiten 4347-4351, ISSN: 0008-5472
- DATABASE BIOSIS [Online] BIOSCIENCES INFORMATION SERVICE, PHILADELPHIA, PA, US; 1996 DENCE CARMEN S ET AL: "Carbon-11-labeled estrogens as potential imaging agents for breast tumors." Database accession no. PREV199699090520 XP002166907 & NUCLEAR MEDICINE AND BIOLOGY, Bd. 23, Nr. 4, 1996, Seiten 491-496, ISSN: 0969-8051
- DATABASE BIOSIS [Online] BIOSCIENCES INFORMATION SERVICE, PHILADELPHIA, PA, US; 1997 LOBACCARO CAROLE ET AL: "Steroidal affinity labels of the estrogen receptor: 3. Estradiol 11-beta-n-alkyl derivatives bearing a terminal electrophilic group: Antiestrogenic and cytotoxic properties." Database accession no. PREV199799646919 XP002166908 & JOURNAL OF MEDICINAL CHEMISTRY, Bd. 40, Nr. 14, 1997, Seiten 2217-2227, ISSN: 0022-2623
- NAPOLITANO ET AL: "11.beta.-Substituted Estradiol Derivatives, Potential High-Affinity Carbon-11-Labeled Probes for the Estrogen Receptor: A Structure-Affinity Relationship Study" JOURNAL OF MEDICINAL CHEMISTRY,AMERICAN CHEMICAL SOCIETY. WASHINGTON,US, Bd. 38, Nr. 3, 1995, Seiten 429-434, XP002149421 ISSN: 0022-2623
- TEDESCO R ET AL: "7alpha,11beta-disubstituted estrogens: probes for the shape of the ligand binding pocket in the estrogen receptor" BIOORGANIC & MEDICINAL CHEMISTRY LETTERS,GB,OXFORD, Bd. 7, Nr. 22, 18. November 1997 (1997-11-18), Seiten 2919-2924, XP004136557 ISSN: 0960-894X
- ANSTEAD G M ET AL: "The estradiol pharmacophore: Ligand structure-estrogen receptor binding affinity relationships and a model for the receptor binding site" STEROIDS: STRUCTURE, FUNCTION, AND REGULATION,US,ELSEVIER SCIENCE PUBLISHERS, NEW YORK, NY, Bd. 62, Nr. 3, 1. März 1997 (1997-03-01), Seiten 268-303, XP004057108 ISSN: 0039-128X
- R B GABBARD ET AL: "STRUCTURE ACTIVITY RELATIONSHIPS OF ESTROGENS. EFECTS OF 14-DEHYDROGENATION AND AXIAL METHYL GROUPS AT C-7, C-9 AND C-11" STEROIDS: STRUCTURE, FUNCTION, AND REGULATION,US,ELSEVIER SCIENCE PUBLISHERS, NEW YORK, NY, Bd. 41, Nr. 6, Juni 1983 (1983-06), Seiten 791-805, XP002137796 ISSN: 0039-128X

## Beschreibung

### Feld der Erfindung

Die vorliegende Erfindung bezieht sich auf neue Verbindungen als pharmazeutische Wirkstoffe, die in vitro eine höhere Affinität an Estrogenrezeptorpräparationen von Rattenprostata als an Estrogenrezeptorpräparationen von Rattenuterus und in vivo eine präferentielle Wirkung am Knochen im Vergleich zum Uterus und/oder ausgeprägte Wirkung hinsichtlich Stimulierung der Expression von 5HT2a-Rezeptor und -transporter aufweisen, deren Herstellung, ihre therapeutische Anwendung und pharmazeutischen Darreichungsformen, die die neuen Verbindungen enthalten.
Bei den chemischen Verbindungen handelt es sich um neuartige steroidale gewebeselektive Estrogene.

### Hintergrund der Erfindung

Etablierte Estrogentherapien zur Behandlung von hormondefizienzbedingten Beschwerden und die protektive Wirkung von Estrogenen auf Knochen, Gehirn, Gefäß und andere Organsysteme.

Die Effizienz von Estrogenen in der Behandlung von hormondefizienzbedingten Symptomen wie Hitzewallungen, Atrophie von Estrogenzielorganen und Inkontinenz, sowie die erfolgreiche Anwendung von Estrogen-Therapien zur Verhinderung von Knochenmasseverlust bei peri- und postmenopausalen Frauen, ist gut belegt und allgemein akzeptiert (Grady et al.1992, Ann Intern Med 117: 1016-1037). Ebenso ist gut dokumentiert, daß die Estrogenersatztherapie bei postmenopausalen Frauen oder bei Frauen mit anders bedingter ovarieller Dysfunktion, das Risiko von Herzkreislauferkrankungen gegenüber nicht estrogenbehandelten Frauen reduziert (Grady et al., loc. cit.).
Neuere Untersuchungen belegen zudem eine protektive Wirkung von Estrogenen gegen neurodegenerative Erkrankungen, wie z.B. Alzheimersche Krankheit (Henderson 1997, Neurology 48 (Suppl 7): S27-S35; Birge 1997, Neurology 48 (Suppl 7): S36-S41), eine schützende Wirkung auf Gehirnfunktionen, wie Gedächtnisleistung und Lernfähigkeit (McEwen et al. 1997, Neurology 48 (Suppl 7): S8-S15; Sherwin 1997, Neurology 48 (Suppl 7): S21-S26), sowie gegen hormondeFziensbedingte Stimmungsschwankungen (Halbreich 1997, Neurology 48 (Suppl 7): S16-S20).

Weiterhin hat sich Estrogenersatztherapie als effektiv hinsichtlich der Reduktion der Inzidenz von Kolonrektalkarzinom erwiesen (Calle EF et al., 1995, J Natl Cancer Inst 87: 517-523).

In der herkömmlichen Estrogen- oder Hormonersatztherapie (Hormone Replacement Therapy = HRT) werden natürliche Estrogene, wie Estradiol und konjugierte Estrogene aus Pferdeurin entweder allein oder in Kombination mit einem Gestagen eingesetzt. Anstelle der natürlichen Estrogene können auch durch Veresterung erhaltene Derivate, wie z.B. das 17β-Estradiolvalerat, eingesetzt werden.
Wegen der stimulierenden Wirkung der verwendeten Estrogene auf das Endometrium, die zu einer Erhöhung des Endometriumkarzinomrisikos führt (Harlap S 1992, Am J Obstet Gynecol 166: 1986-1992), werden in der Hormonersatztherapie vorzugsweise Estrogen/Gestagen-Kombinationspräparate eingesetzt. Die gestagene Komponente in der Estrogen/Gestagen-Kombination vermeidet eine Hypertrophie des Endometriums, allerdings ist mit der gestagenhaltigen Kombination auch das Auftreten ungewünschter Zwischenblutungen verknüpft.

Eine neuere Alternative zu den Estrogen/Gestagen-Kombinationspräparaten stellen selektive Estrogene dar. Bisher werden unter selektiven Estrogenen solche Verbindungen verstanden, die estrogenartig auf Gehirn, Knochen und Gefäßsystem, aufgrund ihrer antiuterotrophen (d.h. antiestrogenen) Partialwirkung aber nicht proliferativ auf das Endometrium wirken.

Eine Klasse von Substanzen, die das gewünschte Profil eines selektiven Estrogens teilweise erfüllen, sind die sogenannten ,Selective Estrogen Receptor Modulators' (SERM) (R.F. Kauffman, H.U. Bryant 1995, DNAP 8 (9): 531-539). Es handelt sich hierbei um Partialagonisten des Estrogenrezeptorsubtyps ,ERα'. Dieser Typ von Substanzen ist allerdings ineffektiv hinsichtlich der Therapie akuter postmenopausaler Beschwerden, wie z.B. Hitzewallungen. Als Beispiel für ein SERM sei das kürzlich für die Indikation Osteoporose eingeführte Raloxifen genannt.

### Estrogenrezeptor beta (ERβ)

Kürzlich wurde der Estrogenrezeptor-β (ERβ) als zweiter Subtyp des Estrogenrezeptors entdeckt (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). Das Expressionsmuster von ERβ unterscheidet sich von dem des ERα (Kuiper et al. (1996), Endocrinology 138: 863-870). So überwiegt ERβ gegenüber ERα in der Rattenprostata, während in Rattenuterus ERα gegenüber ERβ überwiegt. Im Gehirn wurden Areale identifiziert, in denen jeweils nur einer der beiden ER-Subtypen exprimiert wird (Shugrue et al. (1996), Steroids 61: 678-681; Li et al. (1997), Neuroendocrinology 66:63-67). ERβ wird u.a. in Arealen exprimiert, denen Bedeutung für kognitive Prozesse und ,Stimmung' zugewiesen wird (Shugrue et al. 1997, J Comparative Neurology 388:507-525).

Molekulare Targets für ERβ in diesen Gehirnarealen könnten der 5HT2a-Rezeptor und der Serotonintransporter sein (G. Fink & B.E.H. Sumner 1996 Nature 383:306; B. E.H. Sumner et al. 1999 Molecular Brain Research, in press). Der Neurotransmitter Serotonin (5-Hydroxytryptamin) ist an der Regulation einer Vielzahl von Prozessen beteiligt, die in der Menopause beeinträchtigt sein können. Insbesondere die Effekte der Menopause auf Stimmung und Kognition werden mit dem serotonergen System in Verbindung gebracht. Estrogenersatztherapie hat sich als effektiv hinsichtlich Behandlung dieser Estrogendefizienzbedingten Beschwerden erwiesen, möglicherweise durch Modulation von Serotoninrezeptor- und -Transporterexpression.

Weitere Organsysteme mit vergleichsweise hoher ERβ-Expression umfassen den Knochen (Onoe Y et al., 1997, Endocrinology 138:4509-4512), das Gefäßsystem (Register TC, Adams MR 1998, J Steroid Molec Biol 64: 187-191), den Urogenitaltrakt (Kuiper GJM et al. 1997, Endocrinology 138: 863-870), den Gastrointestinaltrakt (Campbell-Thopson 1997, BBRC 240: 478-483), sowie die Testis (Mosselmann S et al. 1996 Febs Lett 392 49-53) einschließlich der Spermatiden (Shugrue et al. 1998, Steroids 63: 498-504). Die Gewebeverteilung legt nahe, daß Estrogene über ERβ Organfunktionen regulieren. Daß ERβ in dieser Hinsicht funktionell ist, ergibt sich auch durch Untersuchungen an ERα- (ERKO) bzw. ERβ-(βERKO)-Knockout-Mäusen:Ovariektomie bewirkt Knochenmasseverlust in ERKO-Mäusen, der durch Estrogensubstitution aufgehoben werden kann (Kimbro et al. 1998, Abstract OR7-4, Endocrine Society Meeting New Orleans). Ebenso hemmt Estradiol in Blutgefäßen weiblicher ERKO-Mäuse die Gefäßmedia- und Glattmuskelzellproliferation (Iafrati MD et al. 1997, Nature Medicine 3: 545-548). Diese protektiven Wirkungen von Estradiol erfolgen in der ERKO-Maus vermutlich über ERβ.
Beobachtungen an βERKO-Mäusen liefern einen Hinweis auf eine Funktion von ERβ in Prostata und Blase: bei älteren männlichen Mäusen treten Symptome von Prostata- und Blasenhyperplasie auf (Krege JH et al. 1998, Proc Natl Acad Sci 95: 15677-15682). Außerdem weisen weibliche (Lubahn DB et al. 1993, Proc Natl Acad Sci 90:11162-11166) und männliche ERKO-Mäuse (Hess RA et al. 1997, Nature 390: 509-512) sowie weibliche βERKO-Mäuse (Krege JH, 1998) Fertilitätsstörungen auf. Hierdurch wird die wichtige Funktion von Estrogenen hinsichtlich Aufrechterhaltung von Testis- und Ovarfunktion sowie Fertilität belegt.

Eine selektive Estrogenwirkung auf bestimmte Zielorgane könnte aufgrund der unterschiedlichen Gewebe- bzw. Organverteilungverteilung der beiden Subtypen des ERs durch subtypspezifische Liganden erreicht werden. Substanzen mit Präferenz für ERβ verglichen mit ERα im in vitro Rezeptorbindungstest wurden von Kuiper et al. beschrieben (Kuiper et al. (1996), Endocrinology 138: 863-870). Eine selektive Wirkung von subtypspezifischen Liganden des Estrogenrezeptors auf estrogensensitive Parameter in vivo wurde bisher nicht gezeigt.

Aufgabe der vorliegenden Erfindung ist es deshalb, Verbindungen bereitzustellen, die in vitro eine Dissoziation hinsichtlich Bindung an Estrogenrezeptorpräparationen von Rattenprostata und Rattenuterus und die in vivo eine Dissoziation hinsichtlich Knochen- im Vergleich zur Uteruswirkung aufweisen. Die Verbindungen sollen in vitro eine höhere Affinität an Estrogenrezeptorpräparationen von Rattenprostata als an Estrogenrezeptorpräparationen von Rattenuterus und in vivo eine höhere Potenz hinsichtlich Protektion gegen hormondefizienzbedingten Knochenmasseverlust im Vergleich zur uterusstimulierenden Wirkung Uterus und/oder ausgeprägte Wirkung hinsichtlich Stimulierung der Expression von 5HT2a-Rezeptor und -transporter aufweisen.

Im weiteren Sinne soll durch die vorliegende Erfindung eine Struktur-Wirkungsbeziehung zur Verfügung gestellt werden, die den Zugang zu Verbindungen gestattet, die das oben formulierte pharmakologische Profil, bessere estrogene Wirkung am Knochen als am Uterus, besitzen.

Erfindungsgemäß gelöst wird die vorstehende Aufgabe durch die Bereitstellung der Gona-1,3,5(10)-trienderivate der allgemeinen Formel I' 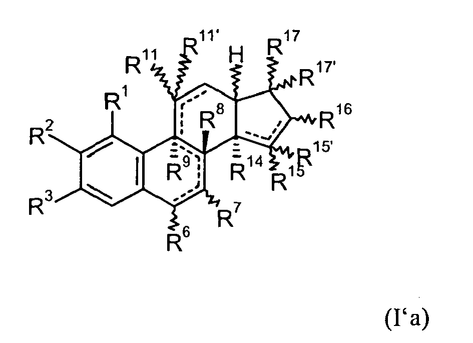 worin
- R¹: ein Halogenatom, ein Rest R¹⁸- oder R¹⁸-O-, wobei R¹⁸ ein Wasserstoffatom oder einen gerad- oder verzweigtkettigen, gesättigten oder ungesättigten Kohlenwasserstoffrest mit bis zu 6 Kohlenstoffatomen, eine Trifluormethylgruppe,
- R²: ein Halogenatom;
ein Rest R¹⁸- oder R¹⁸-O-, wobei R¹⁸ die unter R¹ angegebene Bedeutung hat; eine Gruppe R¹⁹SO₂-O-, worin R¹⁹ eine R²⁰R²¹N - Gruppe ist, wobei R²⁰ und R²¹ unabhängig voneinander ein Wasserstoffatom, einen C₁ - C₅ - Alkylrest, eine Gruppe C(O)R²², worin R²² einen gerad- oder verzweigtkettigen Kohlenwasserstoffrest mit bis zu 12 Kohlenstoffatom, der außerdem bis zu drei Doppel- und/oder Dreifachbindungen enthalten kann, einen C₃ - C₇ - Cycloalkylrest, einen Arylrest oder eine Kombination aus diesen Strukturmerkmalen darstellt, oder, zusammen mit dem N-Atom, einen Polymethyleniminorest mit 4 bis 6 C-Atomen oder einen Morpholinorest, bedeuten;
- R³: eine Gruppe R¹⁸-O-, R¹⁹SO₂-O- oder -O-C(O)R²², mit R¹⁸, R¹⁹ und R²² jeweils in der unter R¹ bzw. R² angegebenen Bedeutung, wobei für R¹⁸ zusätzlich ein Aryl-, Heteroaryl- oder Aralkylrest stehen kann;
- R⁶ und R⁷: unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R¹ bzw. R² angegebenen Bedeutung, eine gerad- oder verzweigtkettige, gesättigte oder ungesättigte, teilweise oder vollständig halogenierte Alkylgruppe mit bis zu 10 Kohlenstoffatomen oder ein gegebenenfalls substituierter Aryl-, Heteroaryl- oder Aralkylrest,
- R⁸ und R⁹: unabhängig voneinander ein Wasserstoffatom, einen gerad- oder verzweigtkettigen, gesättigten oder ungesättigten, gegebenenfalls teilweise oder vollständig halogenierten Kohlenwasserstoffrest mit bis zu 10 Kohlenstoffatomen, eine Gruppe -X- R¹⁸, worin X ein Sauerstoff- oder Schwefelatom ist und R¹⁸ die unter R¹ angegebene Bedeutung hat, ein Halogenatom, eine Cyano-oder Rhodanogruppe,
- R¹¹: ein Wasserstoffatom, ein Halogenatom, eine Gruppe, R¹⁹SO₂-O- oder - R²², mit R¹⁸, R¹⁹ und R²² jeweils in der unter R¹ bzw. R² angegebenen Bedeutung, eine Gruppe -X- R¹⁸, worin X ein Sauerstoff- oder Schwefelatom ist und R¹⁸ die unter R¹ angegebene Bedeutung hat, eine Nitrooxygruppe, einen gerad- oder verzweigtkettigen, gesättigten oder ungesättigten, teilweise oder vollständig halogenierten Kohlenwasserstoffrest mit bis zu 10 Kohlenstoffatomen oder ein gegebenenfalls substituierter Aryl-, Heteroaryl- oder Aralkylrest, sowie
- R^{11'}: ein Wasserstoffatom oder
- R¹¹ und R^{11'}: gemeinsam eine Methylengruppe
- R¹⁴: ein α-ständiges Wasserstoffatom oder eine gerad- oder verzweigtkettige Alkyl-, Alkenyl- oder Alkinylgruppe mit bis zu 3 Kohlenstoffatomen und
- R¹⁵: ein Wasserstoffatom
oder
- R¹⁴ und R¹⁵: zusammen eine gegebenenfalls ein- oder zweifach halogenierte Methylengruppe;
- R^{15'} und R¹⁶: unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R¹ bzw. R² angegebenen Bedeutung;
- R¹⁷ und R^{17'}: ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²², mit R¹⁸, R¹⁹ und R²² jeweils in der unter R¹ bzw. R² angegebenen Bedeutung; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R¹ bzw. R² angegebenen Bedeutung; oder
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder ein Sauerstoffatom;
bedeuten und
in den Positionen 6, 7; 7, 8; 9, 11; 11, 12; 14, 15 sowie 15, 16 eine oder mehrere Doppelbindungen oder 2 Doppelbindungen in den Positionen 6, 7 und 8, 9 vorhanden sein können zur Herstellung eines Arzneimittels zur Behandlung von peri- und postmenopausalen Beschwerden.

Die möglichen Substituenten an den Kohlenstoffatomen 6, 7, 11, 15, 16 und 17 sowie das Wasserstoffatom am Kohlenstoffatom 13 können jeweils in der α- oder β-Position stehen.

Gemäß einer Variante der Erfindung werden vorzugsweise Verbindungen der allgemeinen Formel I' verwendet,
worin
- R¹: ein Wasserstoffatom oder eine Gruppe R¹⁸-O-, wobei R¹⁸ ein Wasserstoffatom oder einen gerad- oder verzweigtkettigen, gesättigten oder ungesättigten Kohlenwasserstoffrest mit bis zu 6 Kohlenstoffatomen,
- R²: ein Wasserstoff- oder Halogenatom oder eine Hydroxygruppe
- R³: eine Gruppe R¹⁸-O-, R¹⁹SO₂-O- oder -O-C(O)R²², mit R¹⁸, R¹⁹ und R²² jeweils in der unter R¹ bzw. R² angegebenen Bedeutung, wobei für R¹⁸ zusätzlich ein Aryl- oder Aralkylrest stehen kann;
- R⁶: ein Wasserstoffatom, ein Hydroxygruppe, eine Gruppe R²² in der unter R² angegebenen Bedeutung ein gegebenenfalls substituierter Aryl-, Heteroaryl- oder Aralkylrest,
- R⁷: ein Wasserstoffatom, ein Halogenatom, eine Gruppe R¹⁸-O-, R¹⁹SO₂-O- oder - R²², mit R¹⁸, R¹⁹ und R²² jeweils in der unter R¹ bzw. R² angegebenen Bedeutung, eine gerad- oder verzweigtkettige, gesättigte oder ungesättigte, teilweise oder vollständig halogenierte Alkylgruppe mit bis zu 10 Kohlenstoffatomen oder ein gegebenenfalls substituierter Aryl-, Heteroaryl- oder Aralkylrest,
- R⁸ und R⁹: unabhängig voneinander ein Wasserstoffatom, einen gerad- oder verzweigtkettigen, gesättigten oder ungesättigten, gegebenenfalls teilweise oder vollständig halogenierten Kohlenwasserstoffrest mit bis zu 10 Kohlenstoffatomen, eine Gruppe -X- R¹⁸, worin X ein Sauerstoff- oder Schwefelatom ist und R¹⁸ die unter R¹ angegebene Bedeutung hat, ein Halogenatom, eine Cyano-oder Rhodanogruppe,
- R¹¹: ein Wasserstoffatom, ein Halogenatom, eine Gruppe -R²² mit R²² in der unter R² angegebenen Bedeutung, eine Gruppe -X- R¹⁸, worin X ein Schwefelatom ist und R¹⁸ die unter R¹ angegebene Bedeutung hat, einen gerad- oder verzweigtkettigen, gesättigten oder ungesättigten, teilweise oder vollständig halogenierten Kohlenwasserstoffrest mit bis zu 10 Kohlenstoffatomen oder ein gegebenenfalls substituierter Aryl- oder Heteroarylrest,
- R¹⁴: ein α-ständiges Wasserstoffatom oder eine gerad- oder verzweigtkettige Alkinylgruppe mit bis zu 3 Kohlenstoffatomen und
- R15: ein Wasserstoffatom
oder
- R¹⁴ und R¹⁵: zusammen eine Methylengruppe;
- R^{15'} und R¹⁶: unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R¹ bzw. R² angegebenen Bedeutung;
- R¹⁷ und R¹⁷': ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²², mit R¹⁸, R¹⁹ und R²² jeweils in der unter R¹ bzw. R² angegebenen Bedeutung; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R¹ bzw. R² angegebenen Bedeutung; sowie
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder ein Sauerstoffatom;
bedeuten.

Eine weitere bevorzugte Variante der vorliegenden Erfindung sieht die Verwendung solcher Verbindungen der allgemeinen Formel I' vor,
worin
- R¹: ein Wasserstoffatom, eine Hydroxy- oder gerad- oder verzweigtkettige C₁-C₆-Alkylgruppe,
- R²: ein Wasserstoff- oder Fluoratom oder eine Hydroxygruppe,
- R³: eine Gruppe R¹⁸-O-, R¹⁹SO₂-O- oder -O-C(O)R²², mit R¹⁸, R¹⁹ und R²² jeweils in der unter R¹ bzw. R² angegebenen Bedeutung, wobei für R¹⁸ zusätzlich ein Aryl- oder Aralkylrest stehen kann;
- R⁶: ein Wasserstoffatom oder eine Hydroxygruppe,
- R⁷: ein Wasserstoffatom, ein Fluor- oder Chloratom, eine Gruppe R¹⁸-O-, R¹⁹SO₂-O- oder -R²², mit R¹⁸, R¹⁹ und R²² jeweils in der unter R¹ bzw. R² angegebenen Bedeutung, eine gerad- oder verzweigtkettige, gesättigte oder ungesättigte, teilweise oder vollständig halogenierte Alkylgruppe mit bis zu 10 Kohlenstoffatomen oder ein gegebenenfalls substituierter Aryl-, Heteroaryl- oder Aralkylrest,
- R⁸ und R⁹: unabhängig voneinander ein Wasserstoffatom, einen gerad- oder verzweigtkettigen, gesättigten oder ungesättigten, gegebenenfalls teilweise oder vollständig halogenierten Kohlenwasserstoffrest mit bis zu 10 Kohlenstoffatomen, eine Gruppe -X- R¹⁸, worin X ein Sauerstoffatom ist und R¹⁸ die unter R¹ angegebene Bedeutung hat, einFluor- oder Chloratom oder eine Cyanogruppe,
- R¹¹: ein Wasserstoffatom, ein Fluor- oder Chloratom, eine gesättigte, gerad- oder verzweigtkettige C₁-C₆-Alkylgruppe, eine Gruppe -X- R¹⁸, worin X ein Schwefelatom ist und R¹⁸ eine gesättigte, gerad- oder verzweigtkettige C₁-C₆-Alkylgruppe, eine Chormethyl- oder Chlorethylgruppe oder ein gegebenenfalls substituierter Aryl- oder Heteroarylrest,
- R¹⁴: ein α-ständiges Wasserstoffatom und
- R¹⁵: ein Wasserstoffatom
oder
- R¹⁴ und R¹⁵: zusammen eine Methylengruppe;
- R^{15'} und R¹⁶: unabhängig voneinander ein Wasserstoffatom, ein Fluor- oder Chloratom oder eine Gruppe R¹⁸-O oder -R²², mit R¹⁸ und R²² jeweils in der unter R¹ bzw. R² angegebenen Bedeutung;
- R¹⁷ und R^{17'}: ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²², mit R¹⁸, R¹⁹ und R²² jeweils in der unter R¹ bzw. R² angegebenen Bedeutung; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R¹ bzw. R² angegebenen Bedeutung; sowie
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder ein Sauerstoffatom;
bedeuten

Gemäß einer weiteren Variante kommen Gona-1,3,5(10)-trien-derivate der allgemeinen Formel I' zur Verwendung,
worin

R⁶, R⁸, R⁹, R¹⁴, R¹⁵, R^{15'} und R¹⁶ jeweils für ein Wasserstoffatom stehen und alle anderen Substituenten die in Anspruch 2 angegebenen Bedeutungen haben.

Wenn die Gonatrien-derivate der allgemeinen Formel I' weitere Doppelbindungen im B-, C- und/oder D-Ring enthalten, dann ist vorzugsweise in der Position 7, 8 oder in der Position 11, 12 eine Doppelbindung oder sind in den Positionen 6, 7 und 8, 9 zwei Doppelbindungen vorhanden.

Eine weitere Variante der Erfindung sind Verwendungen der Gonatrien-derivate der allgemeinen Formel I'a
worin
R¹⁷ und R^{17'} eine Gruppe R¹⁸-O- und eine Gruppe R¹⁸-; eine Gruppe R¹⁸- und eine Gruppe-O-C(O)R²², mit R¹⁸ und R²² jeweils in der unter R¹ bzw. R² angegebenen Bedeutung;
ist.

Von diesen letztgenannten sind wiederum solche Gonatrien-derivate bevorzugt
worin
R¹⁷ und R^{17'} eine Hydroxygruppe und ein Wasserstoffatom, eine C₁ - C₄-Alkyl- oder C₂ - C₄-Alkenylgruppe
ist
und insbesondere bevorzugt diejenigen
worin
R¹⁷ und R^{17'} eine Hydroxygruppe und ein Wasserstoffatom, eine Methyl-, Ethinyl- oder Prop-1-inylgruppe
ist.

In den Verbindungen der allgemeinen Formeln I'a kann für ein Halogenatom immer ein Fluor-, Chlor-, Brom- oder Iodatom stehen; ein Fluoratom ist jeweils bevorzugt. Für die 11β-Position ist insbesondere auch ein Chloratom als Substituent zu nennen. Insbesondere handelt es sich bei den Kohlenwasserstoffresten, die teilweise oder vollständig halogeniert sein können, um fluorierte Reste.

Die Alkoxygruppen in den Verbindungen der allgemeinen Formeln I'a können jeweils 1 bis 6 Kohlenstoffatome enthalten, wobei Methoxy-, Ethoxy- Propoxy- Isopropoxy- und t-Butyloxygruppen bevorzugt sind.

Als Vertreter für die Alkylthiogruppen seien beispielsweise die Methylthio-, Ethylthio- und Trifluormethylthiogruppe genannt.

Beim einem Arylrest handelt es sich im Sinne der vorliegenden Erfindung um einen Phenyl-, 1- oder 2-Naphthylrest; der Phenylrest ist bevorzugt.

Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, erwähnt.

Als Vertreter für gerad- oder verzweigtkettige Kohlenwasserstoffreste mit 1 bis max. 12 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl, Decyl, Undecyl und Dodecyl zu nennen; Methyl, Ethyl, Propyl und Isopropyl sind bevorzugt.

Als C₃-C₇-Cycloalkylgruppe ist eine Cyclopropyl-, butyl-, pentyl-, hexyl- oder heptylgruppe zu nennen.

Bei einem Aralkylrest handelt es sich um einen Rest, der im Ring bis 14, bevorzugt 6 bis 10, C-Atome und in der Alkylkette 1 bis 8, bevorzugt 1 bis 4, C-Atome enthält. So kommen als Aralkylreste beispielsweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H CO₂-Alkyl, -NO₂, -N₃, -CN, C₁-C₂₀-Alkyl, C₁-C₂₀-Acyl, C₁-C₂₀-Acyloxy-Gruppen.

Die Alkylgruppen können teilweise oder vollständig fluoriert oder substituiert sein durch 1-5 Halogenatome, Hydroxygruppen oder C₁-C₄-Alkoxygruppen.

Mit einem C₂-C₃-Alkenyl- bzw. C₂-C₃-Alkinylrest ist ein Vinyl-, oder Allyl- bzw. ein Ethinyl-, 1- oder 2-Propinylrest gemeint.

Als perfluorierte Alkylgruppen seien beispielsweise Trifluormethyl, Pentafluorethyl und Nonafluorbutyl genannt. Vertreter der teilweise fluorierten Alkylgruppen sind zum Beispiel 2,2,2-Trifluorethyl, 5,5,5,4,4-Pentafluorpentyl, 6,6,6,5,5,4,4,3,3-Nonafluorhexyl etc..

Für die halogensubstituierte 14,15-Methylengruppe kann Monochlormethylen, Monofluormethylen oder Difluormethylen stehen.

Weitere Varianten der Erfindung sehen eine oder mehrere, gegebenenfalls konjugierte, Doppelbindungen in den Ringen B, C und D des Estratrien-Gerüsts vor, und zwar eine oder mehrere Doppelbindungen in den Positionen 6, 7; 7, 8; 9, 11; 11, 12; 14, 15 sowie 15, 16. Bevorzugt ist dabei eine Doppelbindung in der Position 7, 8 oder in der Position 11, 12 oder zwei Doppelbindungen in den Positionen 6, 7 und 8, 9 (d.h. zusammen mit dem aromatischen A-Ring wird das Naphthalinsystem ausgebildet).

Eine oder beide Hydroxylgruppen an den C-Atomen 3 und 17 können mit einer aliphatischen, gerad- oder verzweigtkettigen, gesättigten oder ungesättigten C₁-C₁₄-Mono- oder Polycarbonsäure oder einer aromatischen Carbonsäure oder mit einer α- oder β-Aminosäure verestert sein.
Als derartige Carbonsäuren zur Veresterung kommen beispielsweise in Betracht:
Monocarbonsäuren: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure.
Die Veresterung mit Essigsäure, Valeriansäure oder Pivalinsäure ist bevorzugt. Dicarbonsäuren: Oxalsäure, Malonsäure, Bernsteinsäure, Glutarsäure, Adipinsäure, Pimelinsäüre, Korksäure, Azelainsäure, Sebacinsäure, Maleinsäure, Fumarsäure, Muconsäure, Citraconsäure, Mesaconsäure.
Aromatische Carbonsäuren: Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure.
Die Veresterung mit Benzoesäure ist bevorzugt.
Als Aminosäuren kommen die dem Fachmann hinlänglich bekannten Vertreter dieser Substanzklasse in Frage, beispielsweise Alanin, β-Alanin, Arginin, Cystein, Cystin, Glycin, Histidin, Leucin, Isoleucin, Phenylalanin, Prolin etc..
Die Veresterung mit β-Alanin ist bevorzugt.

Bevorzugt gemäß vorliegender Erfindung sind die nachstehenden Verbindungen:
11β-Fluor-gona-1,3,5(10)-trien-3,17-diol
11β-Chlor-gona-1,3,5(10)-trien-3,17-diol
11β-Methyl-gona-1,3,5(10)-trien-3,17-diol
11β-Ethyl-gona-1,3,5(10)-trien-3,17-diol
11β-Phenyl-gona-1,3,5(10)-trien-3,17-diol
7α-Fluor-gona-1,3,5(10)-trien-3,17-diol
7α-Methyl-gona-1,3,5(10)-trien-3,17β-diol
7α-Phenyl-gona-1,3,5(10)-trien-3,17β-diol
7α-Methyl-gona-1,3,5(10)-trien-3,17-diol
7β-Fluor-gona-1,3,5(10)-trien-3,17-diol
7β-Methyl-gona-1,3,5(10)-trien-3,17β-diol
7β-Phenyl-gona-1,3,5(10)-trien-3,17β-diol
7β-Methyl-gona-1,3,5(10)-trien-3,17-diol
7β-Ethyl-gona-1,3,5(10)-trien-3,17β-diol
7β-Ethyl-gona-1,3,5(10)-trien-3,17α-diol
7β-Ethyl-13α-H-gona-1,3,5(10)-trien-3,17α-diol
2-Fluor-gona-1,3,5(10)-trien-3,17β-diol
17α-(1-Propinyl)-gona-1,3,5(10)-trien-3,17β-diol
11-Methylen-17α-(1-propinyl)-gona-1,3,5(10)-trien-3,17β-diol
11β-Methyl-17α-(1-propinyl)-gona-1,3,5(10)-trien-3,17β-diol
11β,17α-Dimethyl-gona-1,3,5(10)-trien-3,17β-diol
11β,17β-Dimethyl-gona-1,3,5(10)-trien-3,17α-diol
13α-H-18-Nor-estradiol
18-Nor-estriol
11β-Fluor-gona-1,3,5(10)-trien-1,3,17-triol
11β-Chlor-gona-1,3,5(10)-trien-1,3,17-triol
11β-Methyl-gona- 1,3,5(1 10)-trien-1,3,17-triol
11β-Ethyl-gona-1,3,5(10)-trien-1,3,17-triol
11β-Phenyl-gona-1,3,5(10)-trien-1,3,17-triol
7α-Fluor-gona-1,3,5(10)-trien-1,3,17-triol
7α-Methyl-gona-1,3,5(10)-trien-1,3,17-triol
7α-Phenyl-gona-1,3,5(10)-trien-1,3,17-triol
7α-Methyl-gona-1,3,5(10)-trien-1,3,17-triol
7β-Fluor-gona-1,3,5(10)-trien-1,3,17-triol
7β-Methyl-gona-1,3,5(10)-trien-1,3,17-triol
7β-Phenyl-gona-1,3,5(10)-trien-1,3,17-triol
7β-Methyl-gona-1,3,5(10)-trien-1,3,17-triol
2-Fluor-gona-1,3,5(10)-trien-1,3,17-triol
17α-(1-Propinyl)-gona-1,3,5(10)-trien-1,3,17,triol
11-Methylen-17α-(1-propinyl)-gona-1,3,5(10)-trien-1,3,17-triol
11β-Methyl-17α-(1-propinyl)-gona-1,3,5(10)-trien-1,3,17-triol
11β,17α-Dimethyl-gona-1,3,5(10)-trien-1,3,17-triol
11β,17β-Dimethyl-gona-1,3,5(10)-trien-1,3,17-triol

Im Rahmen des Verwendungsaspekts der Erfindung ist zusätzlich zu den vorstehend aufgezählten neuen Verbindungen außerdem die Verwendung der bereits bekannten Verbindung 18-Nor-17β-estradiol bevorzugt.

Die erfindungsgemäßen Ester der 18-Nor-Steroide weisen als Prodrugs Vorteile gegenüber den unveresterten Wirkstoffen hinsichtlich ihres Applikationsmodus, ihrer Wirkungsart, Wirkungsstärke und Wirkungsdauer auf.

Pharmakokinetische und pharmakodynamische Vorteile weisen auch die erfindungsgemäßen 18-Nor-Steroid-Sulfamate auf. Diesbezügliche Effekte wurden bereits bei Sulfamaten beschrieben, die sich von Estrogenen mit einer 13-Methylgruppe ableiten (J. Steroid Biochem. Molec. Biol, 55, 395 - 403 (1995); Exp. Opinion Invest. Drugs 7, 575 - 589 (1998)).

In der vorliegenden Patentanmeldung werden Steroide, denen das Gonatrien- (= 18-Norestratrien-) Gerüst zugrunde liegt, zur Behandlung von Estrogenrezeptor β - vermittelten Krankheiten und Zuständen als selektive Estrogene beschrieben, die in vitro Dissoziation hinsichtlich Bindung an Estrogenrezeptorpräparationen von Rattenprostata und Rattenuterus und die in vivo eine Dissoziation hinsichtlich Knochen- im Vergleich zu Uteruswirkung aufweisen: über einen breiten Dosisbereich wirken die Substanzen knochenprotektiv ohne den Uterus zu stimulieren. Im gleichen Dosisbereich ist ihre Leberwirkung gering.

Die Substanzen üben außerdem estrogenartige Wirkung auf das Gefäßsystem und Gehirnfunktionen aus. Substanzen mit höherer Bindung an den Rattenprostata- verglichen mit dem Rattenuterus-Estrogenrezeptor sind potenter hinsichtlich Erhöhung der Expression von Serotoninrezeptor und -transporter, im Vergleich zu ihrem positiven Effekt auf die LH-Ausschüttung. Daher werden Prozesse, an deren Regulation der Neurotransmitter Serotonin beteiligt ist, günstig beeinflußt und die erfindungsgemäßen Verbindungen üben insbesondere auf die Stimmung und Kognition einen günstigen Einfluß aus.

Sie können als Estrogene in dem in der WO 97/45125 beschriebenen Sinne für die Herstellung von Medikamenten zur Beeinflußung der Spiegel von Serotonin bzw. von Serotonin mRNA beim Menschen verwendet werden.

Es wurde gefunden, daß die erfindungsgemäßen Nor-Steroide als selektive Estrogene zur Behandlung verschiedener Zustände und Krankheiten, die durch einen höheren Gehalt an Estrogenrezeptor β als Estrogenrezeptor α im entsprechenden Zielgewebe oder -organ gekennzeichnet sind, geeignet sind.

Die Erfindung betrifft auch pharmazeutische Präparate, enthaltend mindestens eine Verbindung gemäß Anspruch 1 (oder physiologisch verträgliche Additionssalze mit organischen und anorganischen Säuren davon) enthalten und die Verwendung der Verbindungen der allgemeinen Formel I'a zur Herstellung von Arzneimitteln, insbesondere für die nachstehenden Indikationen.

Die Verbindungen können, sowohl nach oraler als auch parenteraler Gabe, für die folgenden Indikationen eingesetzt werden.

Die im vorliegenden Patent beschriebenen neuartigen selektiven Estrogene können als Einzelkomponente in pharmazeutischen Zubereitungen oder in Kombination insbesondere mit Antiestrogenen oder Gestagenen eingesetzt werden. Besonders bevorzugt ist die Kombination der selektiven Estrogene mit ERα-selektiven Antiestrogenen, oder mit Antiestrogenen, die peripherselektiv wirksam sind, d.h. die die Bluthimschranke nicht passieren.

Die Substanzen und die sie enthaltenden Pharmaka sind besonders geeignet für die Behandlung peri- und postmenopausaler Beschwerden insbesondere Hitzewallungen, Schlafstörungen, Reizbarkeit, Stimmungsschwankungen, Inkontinenz, Vaginalatrophie, hormondefizienzbedingte Gemütserkrankungen. Ebenso sind die Substanzen für die Hormonsubstitution und die Therapie von hormondefizienz bedingten Beschwerden bei chirurgisch, medikamentös oder anders bedingter ovarieller Dysfunktion geeignet. Hierzu gehört auch die Vorbeugung gegen den Knochenmasseverlust bei postmenopausalen Frauen, bei hysterektomierten Frauen oder bei Frauen, die mit LHRH-Agonisten oder -Antagonisten behandelt wurden.

Die Verbindungen sind auch zur Linderung der Symptome der Andropause und Menopause, d.h. zur männlichen und weiblichen Hormonersatz-Therapie (HRT), und zwar sowohl zur Prävention als auch zur Behandlung, weiterhin zur Behandlung der mit einer Dysmenorrhoe einhergehenden Beschwerden sowie zur Behandlung der Akne geeignet.

Die Substanzen sind außerdem zur Prophylaxe gegen hormondefizienzbedingten Knochenmasseverlust und Osteoporose, zur Vorbeugung gegen Herzkreislauferkrankungen, insbesondere Gefäßerkrankungen wie Atherosklerose, zur Hemmung der Proliferation der arteriellen Glattmuskelzellen, zur Behandlung des primären pulmonaren Bluthochdrucks und zur Vorbeugung gegen hormondefizienzbedingte neurodegenerative Erkrankungen, wie Alzheimersche Krankheit, sowie hormondefizienzbedingte Beeinträchtigung von Gedächtnis- und Lernfähigkeit, einsetzbar.

Weiterhin sind die Substanzen zur Behandlung von entzündlichen und Erkrankungen des Immunsystems, insbesondere Autoimmunerkrankungen, wie z.B. Rheumatoide Arthritis, einsetzbar.

Außerdem können die Verbindungen zur Behandlung männlicher Fertilitätsstörungen und prostatischer Erkrankungen Verwendung finden..

Die Verbindungen können auch in Kombination mit dem natürlichen Vitamin D3 oder mit Calcitriol-Analoga für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I'a in Verbindung mit Progesteronrezeptor-Antagonisten verwendet werden, und zwar insbesondere zur Verwendung in der Hormonersatz-Therapie und zur Behandlung gynäkologischer Störungen.

Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die zu verabreichende Menge einer Verbindung der allgemeinen Formel I'a schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 10 mg/kg Körpergewicht, vorzugsweise 0,04 µg/kg - 1 mg/kg Körpergewicht, je Tag betragen. Beim Menschen entspricht dies einer Dosis von 0,8 µg bis 800 mg, vorzugsweise 3,2 µg bis 80 mg, täglich.

Eine Dosiseinheit enthält erfindungsgemäß 1,6 µg bis 200 mg einer oder mehrerer Verbindungen der allgemeinen Formel I'a.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I'a beladenen Intravaginal- (z.B. Vaginalringe) oder Intrauterinsystemen (z.B. Pessare, Spiralen, IUSs, Mirena^{®}) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I' auch mit Liposomen verkapselt werden.

### Methoden

### Estrogenrezeptorbindungsstudien

Die Bindungsaffinität der neuen selektiven Estrogene wurde in Kompetitionsexperimenten unter Verwendung von 3H-Estradiol als Ligand an Estrogenrezeptorpräparationen von Rattenprostata und Rattenuterus getestet. Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289).
Die Präparation von Rattenuteruscytosol, sowie der Rezeptortest mit dem ER-haltigen Cytosol wurden prinzipiell durchgeführt wie von Stack und Gorski, 1985, beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Die im vorliegenden Patent beschriebenen Substanzen weisen höhere Bindungsaffinität zu Estrogenrezeptor aus Rattenprostata als zu Estrogenrezeptor aus Rattenuterus auf. Dabei wird davon ausgegangen, daß ERβ gegenüber ERα in der Rattenprostata, in Rattenuterus ERα gegenüber ERβ überwiegt. Tabelle 1 zeigt, daß das Verhältnis der Bindung an Prostata- und Uterusrezeptor qualitativ mit dem Quotient der relativen Bindungsaffinität (RBA) an humanen ERβ und ERα von Ratte (nach Kuiper et al. (1996), Endocrinology 138: 863-870) übereinstimmt (Tabelle 1).

Tabelle 2 zeigt die Ergebnisse für die erfindungsgemäß zu verwendende Verbindung
18-Nor-17β-estradiol (Verbindung A)
sowie für die erfindungsgemäßen Verbindungen
11β,17α-Dimethyl-gona-1,3,5(10)-trien-3,17β-diol (Verbindung B),
11-Methylen-17α-(1-propinyl)-gona-1,3,5(10)-trien-3,17β-diol (Verbindung C),
17α-(1-propinyl)-gona-1,3,5(10)-trien-3,17β-diol (Verbindung D) sowie
11β-Methyl-17α-(1-propinyl)-gona-1,3,5(10)-trien-3,17β-diol (Verbindung E).

Die Verbindungen A, B, C, D und E zeigen eine höhere Bindungsaffinität am Estrogenrezeptor aus Rattenprostata als am Estrogenrezeptor aus Rattenuterus.

Weiterhin wurde die Prädiktivität des 'Prostata-ER versus Uterus-ER-Testssystems' hinsichtlich gewebeselektiver Wirkung durch in vivo Untersuchungen bestätigt. Substanzen mit Präferenz für Prostata-ER sind in vivo hinsichtlich Knochen- und Uteruswirkung zugunsten der Wirkung am Knochen dissoziiert.

### Knochenuntersuchungen

3 Monate alte weibliche Ratten werden ovarektomiert und unmittelbar nach der Operation 28 Tage lang 1 mal täglich mit der Testverbindung behandelt. Die Applikation erfolgt subcutan in Arachisöl/Ethanol. Die Tiere werden am Tag nach der letzten Applikation getötet und Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 4 - 6 mm vom Gelenkkopf der proximalen Tibia durchgeführt.
Durch die Ovarektomie vermindert sich die Dichte des trabekulären Knochens im gemessenen Bereich von ca. 400 mg Ca²⁺/cm³ auf ca. 300 mg Ca²⁺/cm³. Durch die Behandlung mit einer Verbindung der allgemeinen Formel I gemäß vorliegender Erfindung wird der Abbau der Knochendichte verhindert bzw. gehemmt. Gemessen wurde die Knochendichte an der proximalen Tibia.

In vivo spiegelt sich die höhere Bindungsaffinität zu Estrogenrezeptor aus Rattenprostata als zu Estrogenrezeptor aus Rattenuterus in deutlich niedrigeren Mengen der erindungsgemäßen Verbindungen wider, die eine 50%ige Knochenprotektion bewirken im Vergleich zu den Mengen, die eine 50%ige Uterusstimulation bewirken, bezogen auf den Knochenmasseverlust, der in ovarektomierten, unbehandelten weiblichen Ratten 28 Tage nach der Ovarektomie im Unterschied zu sham-oprierten, intakten Tieren meßbar ist.

Die Gefäßwirkung der erfindungsgemäßen Estrogene wird im Modell der ApoE-Knockout-Maus, wie von R. Elhage et al., 1997, beschrieben, ermittelt (Elhage R. et al. 1997, Arteriosclerosis, Thrombosis, and Vascular Biology 17: 2679-2684).

Zum Nachweis der Wirkung von Estrogenen auf die Gehirnfunktion wird die Oxytozinrezeptor mRNA-Expression als Surrogatparameter verwendet (Hrabovszky E et al. 1998, Endocrinology 1339: 2600-2604). Ovariektomierte Ratten werden über 7 Tage mit der Testsubstanz oder Vehikel behandelt (Applikation: subkutan oder oral, 6-mal täglich). Am Tag 7 nach der ersten Applikation werden die Tiere dekapitiert, das Uterusgewicht wird bestimmt und der Oxytozinrezeptor mRNA Spiegel wird mittels in situ Hybridisierung an geeigneten Gehirnschnitten untersucht. Es werden die ED₅₀-Werte hinsichtlich Stimulierung von Utersuswachstum und Induktion der Oxytozinrezeptor mRNA bestimmt.

Eine andere Möglichkeit, die dissoziierte Estrogenwirkung der erfindungsgemäßen Substanzen in vivo nachzuweisen, besteht darin, nach Einmalapplikation der Substanzen bei Ratten Effekte auf die Expression von 5HT2a-Rezeptor- und Serotonintransporter-Protein- und mRNA-Level in ERβ-reichen Gehirnarealen zuvermessen. Vergleichend zum Effekt auf Serotoninrezeptor- und Transporterexpression wird der Effekt auf die LH-Sekretion gemessen. Substanzen mit höherer Bindung an den Rattenprosta- verglichen mit dem Rattenuterusestrogenrezeptor sind potenter hinsichtlich Erhöhung der Expression von Serotoninrezeptor- und transporter, im Vergleich zu ihrem positiven Effekt auf die LH-Ausschüttung. Die Dichte von Serotoninrezeptor und -Transporter wird an Gehirnschnitten mittels radioaktiver Liganden, die entsprechende mRNA mittels in situ Hybridisierung bestimmt. Die Methode ist in der Literatur beschrieben: G. Fink & B.E.H. Sumner 1996 Nature 383:306; B. E.H. Sumner et al. 1999 Molecular Brain Research, in press.

In Übereinstimmung mit ihrer stärkeren Bindung an den Rattenprostata- im Vergleich zum Rattenuterus-Estrogenrezeptor führen die erfindungsgemäßen Substanzen A, B, C, D und E zu einer erhöhten Expression des Serotoninrezeptors und-transporters.

### Herstellung der erfindungsgemäßen Verbindungen

Zur Herstellung von Gonatrienen (= 18-Nor-Estratrienen) gibt es zwei literaturbekannte Synthesewege:
1. Synthese nach Coombs und Pinhey:M.M. Coombs, C.W. Vose, J.C.S.Chem.Comm. **1974,** 602; J.C. Chapman, J.T. Pinhey, Austr.J.Chem. **1974,** 2421; sowie A. Kuhl, H. Karels, W. Kreiser, Helv.Chem. Acta **1999**, 30;
2. Synthese nach Zeelen:M.J. van den Heuvel, C.W. van Bokhoven, H.P. de Jongh, F.J. Zeelen, Recl.Trav.Chim.Pays-Bas 107,**1988**, 331.

### Synthese nach Zeelen

Dieser Syntheseweg ist geeignet für den Aufbau 11β-substituierter 18-Nor-Steroide.

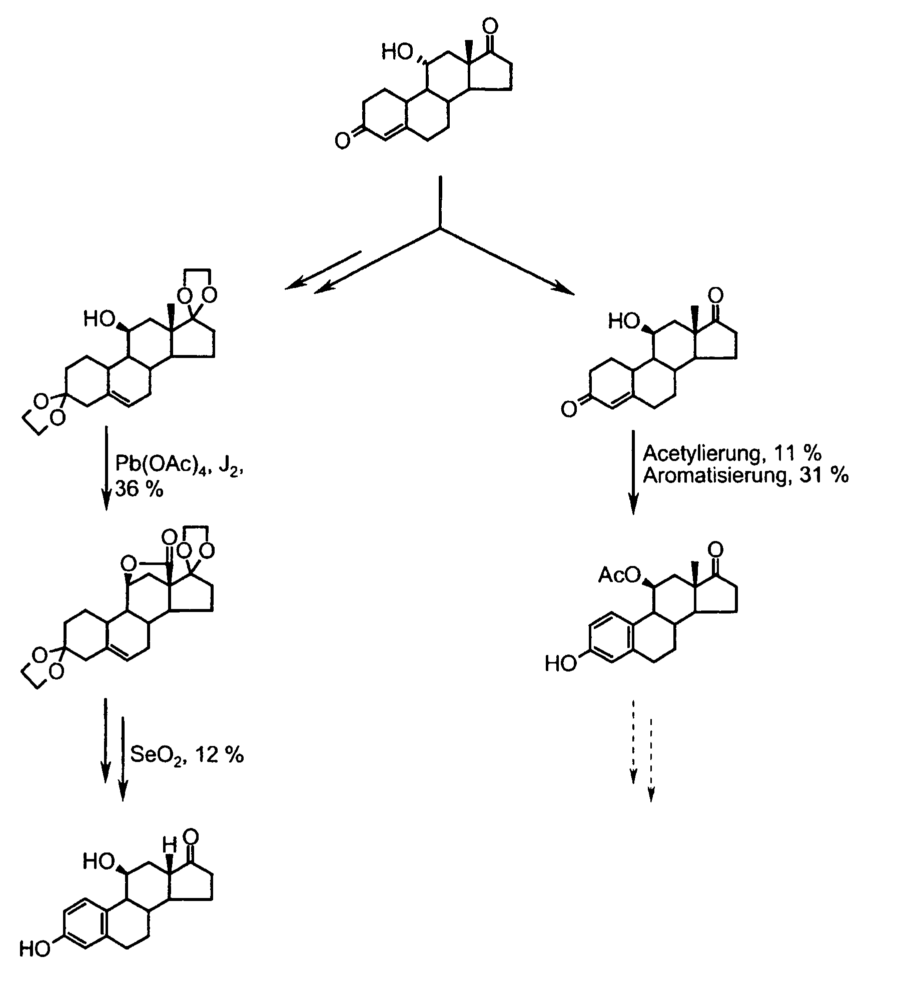

Nach dieser Methode sowie anschließender weiterer Funtionalisierungen an zahlreichen Positionen des Steroidgerüsts lassen sich gemäß DE 195 35 851 A1 gestagene 18-Nor-3-Keto-Δ⁴-Derivate mit einem vielfältigen Substitutionsmuster am Steroid erhalten.
Durch anschließende Aromatisierung der 18-Nor-3-Keto-Δ⁴-Derivate können erfindungsgemäße Verbindungen der allgemeinen Formel I hergestellt werden.
Diese Syntheseroute eignet sich insbesondere für die Herstellung solcher Verbindungen, in denen die 11-Position substituiert ist. Über die 11-Hydroxy- bzw. durch Oxidation daraus zugänglichen 11-Keto-Gruppe lassen sich durch Funktionalisierung, z.B. durch eine Wittig-Reaktion, nach dem Fachmann bekannten Methoden zahlreiche andere Substituenten aufbauen.
Dieses Verfahren wird durch die Beispiele 10 und 11 illustriert.

Gemäß dieser Beispiele wird die Aromatisierung mit Selendioxid durchgeführt.

Genausogut kann die Aromatisierung auch mit dem Fachmann bekannten mikrobiologischen Verfahren bewerkstelligt werden.
Ein Stammscreening ergab, daß die folgenden Mikroorganismen die Reaktion durchführen können:
- ***Bacillus lentus***: ATCC 13805
- ***Corynebacterium simplex***: ATCC 6946
- ***Nocardia corallina***: ATCC 14350
- ***Nocardia globerula***: ATCC 9356

Die besten Ergebnisse lassen sich mit dem Stamm *Bacillus lentus* ATCC 13805 erzielen. Dieser Stamm wurde deshalb für die präparativen Umsetzungen eingesetzt.

### Zur Illustration dienen Beispiele 12 bis 14.

Die Reihenfolge der Reaktionsschritte für die Einführung von funktionellen Gruppen am Steroidgerüst, beispielsweise die Einführung einer Seitenkette am Kohlenstoffatom 17 durch nucleophile Addition, und der Aromatisierung können auch vertauscht werden.

### Synthese nach Coombs und Pinhey

Diese Syntheseroute ist nachstehend am Beispiel der Herstellung des unsubstituierten 18-Nor-17β-estradiols wiedergegeben.

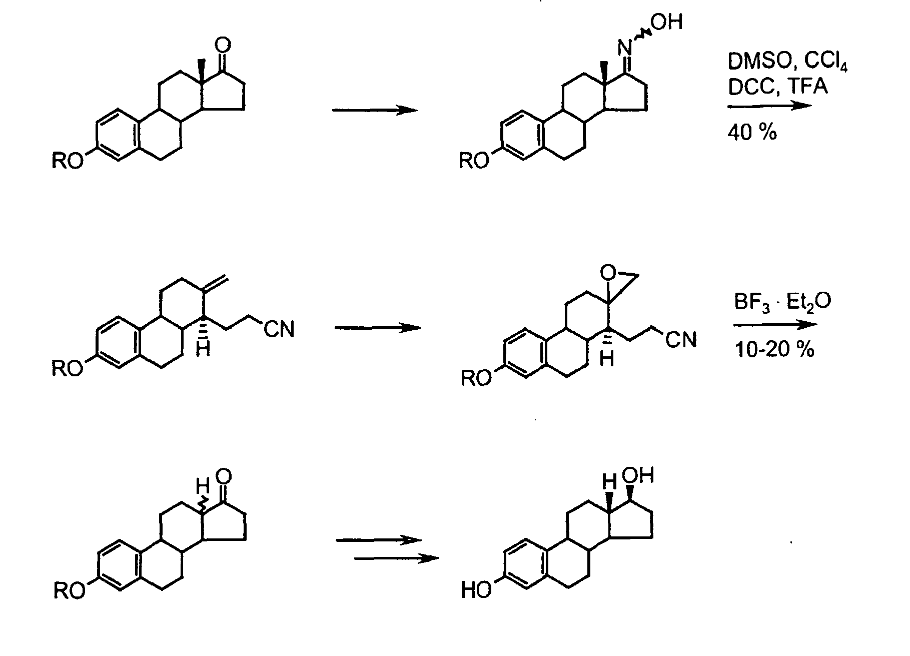

Die Öffnung des D-Rings durch anomale Beckmann-Reaktion läßt sich in etwa 40 % Ausbeute durchführen, die Gesamtausbeute der beiden Folgestufen beträgt jeweils etwa 17 %. Dieser Syntheseweg zur Herstellung der erfindungsgemäßen Verbindungen der allgemeinen Formel I ist in den Beispielen 1 bis 9 illustriert.

Gemäß der allgemeinen Formel I mögliche Substituenten können bereits in der endgültigen Form oder in Form eines Vorläufers schon im Ausgangsprodukt, einem bereits dem gewünschten Endprodukt entsprechend substituierten Estron, vorhanden sein.

So werden Substituenten am Kohlenstoffatom 7 nach dem Fachmann bekannten, beispielsweise auf dem Gebiet der antiestrogenen Wirkstoffe üblichen, Verfahren durch Kupfer-katalysierte 1,6-Addition des Substituenten, oder eines reaktiven Vorläufers davon, an eine 3-Keto-Δ^{4,6}-Verbindung, eingeführt und gegebenenfalls weiter aufgebaut (EP 0 138 504 B, WO 98/07740, WO 99/33855).

Die Einführung eines Substituenten bzw. reaktiven Vorläufers am Kohlenstoffatom 7 ist auch durch nukleophile Addition des Substituenten bzw. Vorläufers an ein 6-Vinylsulfon möglich (DE 42 18 743 A1).
In den beiden letztgenannten Fällen werden in unterschiedlichen Anteilen, abhängig von den Reaktionspartnern und den gewählten Reaktionsbedingungen, 7α- und 7β-substituierte Verbindungen erhalten, die sich beispielsweise durch chromatographische Verfahren trennen lassen.

17-Substituenten werden, ebenfalls nach bekannten Verfahren, durch nukleophile Addition des gewünschten Substituenten oder eines reaktiven Vorläufers davon, eingeführt und gegebebenenfalls weiter aufgebaut.

Substituenten gemäß der allgemeinen Formel I können aber auch auf der Stufe der 18-Nor-Steroide eingeführt werden. Dies kann insbesondere bei Mehrfachsubstitution der gewünschten Endverbindung sinnvoll bzw. erforderlich sein.

Beispielsweise kann eine 11α-Hydroxygruppe nach dem von Vorbrüggen et al. beschriebenen Verfahren in ein 11β-Fluoratom überführt werden.

Funktionalisierungen am Kohlenstoffatom 2 sind beispielsweise durch elektrophile Substitution nach vorheriger Deprotonierung der Position 2 des entsprechenden 3-(2-Tetrahydropyranyl)- oder 3-Methylethers mit einer Lithium-Base (z. B. Methyllithium, Butyllithium) möglich. So kann zum Beispiel ein Fluoratom durch Umsetzung des C-H-aktivierten Substrats mit einem Flourierungsreagenz wie N-Fluormethansulfonimid (WO 94/24098) eingeführt werden.

Die Einführung variabler Substituenten in die Ringe B, C und D des Gonatriengerüstes kann dabei nach der gleichen chemischen Lehre erfolgen, mit der die entsprechenden Estratrienderivate hergestellt werden (siehe unter anderem: Steroide, L.F. Fieser, M. Fieser, Verlag Chemie, Weinheim / Bergstr., 1961; Organic Reactions in Steroid Chemistry, J. Fried, J.A. Edwards, Van Nostrand Reinhold Company, New York, Cincinnati, Toronto, London, Melbourne, 1972; Medicinal Chemistry of Steroids, F.J. Zeelen, Elsevier, Amsterdam, Oxford, New York, Tokyo, 1990). Das betrifft beispielsweise die Einführung von Substituenten, wie Hydroxyl- oder Alkyloxygruppen, Alkyl, Alkenyl- oder Alkinylgruppen oder Halogen, insbesondere Fluor.

Die erfindungsgemäßen 18-Nor-Steroid-Carbonsäureester werden in Analogie zu den Estern hergestellt, die sich von natürlichen Steroidwirkstoffen ableiten (siehe z.B. Pharmazeutische Wirkstoffe, Synthesen, Patente, Anwendungen; A. Kleemann, J. Engel', Georg Thieme Verlag Stuttgart 1978. Arzneimittel, Fortschritte 1972 bis 1985; A. Kleemann, E. Lindner, J. Engel (Hrsg.), VCH 1987, S. 773-814).

Die erfindungsgemäßen 18-Nor-Steroid - Sulfamate sind in an sich bekannter Weise aus den entsprechenden Hydroxy-Steroiden durch Veresterung mit Sulfamoylchloriden in Gegenwart einer Base zugänglich (Z. Chem. **15,** 270-272 (1975); Steroids **61,** 710 - 717 (1996)). Nachfolgende Acylierung der Sulfamidgruppe führt zu den erfindungsgemäßen 18-norsteroidalen (N-Acyl)sulfamaten, für die bereits in der 13-Methyl-Reihe pharmakokinetische Vorteile nachgewiesen wurden (vgl. DE 195 40 233 A1).

Die regioselektive Veresterung von polyhydroxylierten Steroiden mit N-substituierten und N-unsubstituierten Sulfamoylchloriden erfolgt nach partiellem Schutz derjenigen Hydroxylgruppen, die unverestert bleiben sollen. Als Schutzgruppen mit hierfür geeigneter selektiver Reaktivität haben sich Silylether erwiesen, da diese unter den Bedingungen der Sulfamatbildung stabil sind und die Sulfamatgruppe intakt bleibt., wenn die Silylether zur Regenerierung der restlichen im Molekül noch enthaltenen Hydroxylgruppe wieder abgespalten werden (Steroids **61,** 710 - 717 (1996)).
Die Herstellung der erfindungsgemäßen Sulfamate mit einer oder mehreren zusätzlichen Hydroxylgruppen im Molekül ist auch dadurch möglich, daß man von geeigneten Hydroxy-Steroidketonen ausgeht. Zunächst werden, je nach Zielstellung, eine oder mehrere vorhandene Hydroxylgruppen einer Sulfamoylierung unterworfen. Dann können die Sulfamatgrupen gegebenenfalls mit einem gewünschten Acylchlorid in Gegenwart einer Base in die betreffenden /N-Acyl)sulfamate überführt werden. Die nunmehr vorliegenden Oxosulfamate oder Oxo-(N-acyl)sulfamate werden durch Reduktion in die entsprechenden Hydroxysulfamate bzw. Hydroxy-(N-acyl)sulfamate umgewandelt (Steroids **61**, 710 - 717 (1996)).

Als geeignete Reduktionsmittel kommen Natriumborhydrid und der Boran-Dimethylsulfid-Komplex in Frage.

Die erfindungsgemäßen Verbindungen der allgemeinen Formel 1 werden wie in den Beispielen beschrieben hergestellt. Durch analoge Vorgehensweise unter Verwendung homologer Reagenzien zu den in den Beispielen beschriebenen Reagenzien lassen sich weitere Verbindungen der allgemeinen Formel I erhalten.

Veretherung und/oder Veresterung freier Hydroxygruppen erfolgt nach dem Fachmann gängigen Methoden.

Die erfindungsgemäßen Verbindungen können an den Kohlenstoffatomen 6, 7, 11, 13, 15, 16 und 17 als α,β-Stereoisomere vorliegen. Bei der Herstellung der Verbindungen gemäß den beschriebenen Verfahren fallen die Verbindungen meist als Gemische der entsprechenden α,β-Isomeren an. Die Gemische lassen sich beispielsweise durch chromatographische Verfahren trennen.

Die nachfolgenden Beispiele dienen der näheren Erläuterung der Erfindung:

### Beispiele

### Beispiel 1 11β-Fluor-gona-1,3,5(10)-trien-3,17-diol

### 1.1 11β-Fluor-1,3,5(10)-estratrien-3-ol-17-on

In 1500 ml Acetonitril suspendiert man 43,55 g 11β-Fluor-4-estren-17-ol-3-on (150 mmol, Tetrahedron Letters **1995,** 2611), gibt 50 g Kupfer-II-bromid zu und rührt bei Raumtemperatur. Nach 16 Stunden gibt man innerhalb von 6 Stunden weiteres Kupfer-II-bromid in drei Portionen (25 g, 12 g, 6 g) zu und rührt zuletzt noch 6 Stunden bei Raumtemperatur. Die Reaktionsmischung wird im Eisbad abgekühlt, mit 500 ml Wasser versetzt und mit Essigester extrahiert. Die organische Phase versetzt man mit wenig Methanol, wäscht sie mit gesättigter Bicarbonatlösung und Kochsalzlösung aus und trocknet mit Natriumsulfat. Nach Einengen kristallisiert die Substanz aus, Ausbeute 30,8 g (71 % d. Th.), Fp 233-234 °C.

### 1.2 11β-Fluor-3-mesyloxy-estra-1,3,5(10)-trien-17-on

Man löst 28,84 g 11β-Fluor-1,3,5(10)-estratrien-3-ol-17-on (100 mmol) in 200 ml Pyridin, kühlt im Eisbad ab, tropft 20 ml Methansulfonsäurechlorid zu und rührt 2 Stunden bei Raumtemperatur. Dann wird in 2,5 Liter Eiswasser eingerührt und nach 2 Stunden Rühren abfiltriert. Der feste Rückstand wird in Dichlormethan gelöst, die Lösung mit 1N-Salzsäure, Wasser und gesättigter Kochsalzlösung ausgeschüttelt und mit Na₂SO₄ getrocknet. Der Rückstand nach dem Abziehen des Lösungsmittels beträgt 37 g, nach Kristallisation aus Methanol erhält man 33,6 g 11β-Fluor-3-mesyloxy-estra-1,3,5(10)-trien-17-on (91 % d. Th.), Fp.

### 1.3 11β-Fluor-3-mesyloxy-17-oximinoestra-1,3,5(10)-trien

Diese Substanz wird in 500 ml Ethanol suspendiert, mit 20 g Hydroxylaminhydrochlorid und 40 g wasserfreiem Natriumacetat versetzt und 2,5 Stunden lang unter Rückfluß erhitzt. Nach dem Abkühlen wird mit Essigester verdünnt, mit Wasser, gesättigter Bicarbonatlösung und Kochsalzlösung gewaschen und die organische Phase mit Natriumsulfat getrocknet und eingedampft. Der Rückstand kristallisiert aus Ethanol, Ausbeute 31,8 g (91 % d. Th.), Fp.

### 1.4 11β-Fluor-3-mesyloxy-13,17-seco-estra-1,3,5(10),13(18)-tetraen-17-nitril

2,3 g 11β-Fluor-3-mesyloxy-17-oximidoestra-1,3,5(10)-trien (6,3 mmol) löst man in 10 ml Dimethylsulfoxid und 10 ml Tetrachlorkohlenstoff, gibt 3,9 g Dicyclohexylcarbodiimid und kühlt im Eisbad ab. Dann werden 0,33 ml Trifluoressigsäure zugetropft und 3 Stunden gerührt, wobei die Temperatur auf +9 °C ansteigt. Nun wird die Reaktionsmischung in Eiswasser gegeben, dreimal mit Dichlormethan ausgeschüttelt, die organische Phase mit Wasser, gesättigter Bicarbonat-lösung und Kochsalzlösung gewaschen, mit Natriumsulfat getrocknet und eingedampft. Der Rückstand von 4,4 g wird an Kieselgel mit einem Hexan-Essigestergemisch chromatografiert, Ausbeute 0,79 g (36 % d. Th.) als farbloses Öl.

### 1.5 13(18)-Epoxy-1β-fluor-3-mesyloxy-13,17-seco-estra-1,3,5(10)-trien-17-nitril

In 10 ml Dichlormethan löst man 256 mg des Seconitrils (0,7 mmol), gibt 500 mg m-Chlorperbenzoesäure (70 %ig) in zwei Portionen zu und rührt 20 Stunden bei Raumtemperatur. Die Mischung wird mit 10 %iger Kaliumjodidlösung, 1 molarer Natriumdithionit, gesättigter Bicarbonat-lösung und Kochsalzlösung gewaschen, mit Natriumsulfat getrocknet und eingedampft. Der Rückstand (280 mg) wird an Kieselgel mit Hexan und Essigester chromatografiert, Ausbeute 131 mg (49 % d. Th.) als farbloses Öl (Isomerengemisch).

### 1.6 11β-Fluor-3-mesyloxy-gona-1,3,5(10)-trien-17-on

Man löst 571 mg des vorstehenden Epoxides (1,5 mmol) in 200 ml Toluol, fügt 1,3 ml Bortrifluoridetherat zu und erhitzt 16 Stunden auf 110 °C. Nach Abkühlen wird die Mischung mit Essigester verdünnt, mit gesättigtem Bicarbonat und Kochsalzlösung gewaschen, mit Natrium-sulfat getrocknet und eingedampft. Der Rückstand von 577 mg wird an Kieselgel mit Hexan und Aceton chromatografiert, Ausbeute 90 mg (17 % d. Th.) als festen Schaum.

### 1.7 11β-Fluor-gona-1,3,5(10)-trien-3,17-diol

Zur Lösung von 90 mg 11β-Fluor-3-mesyloxy-gona-1,3,5(10)-trien-17-on in 10 ml wasserfreiem THF gibt man 200 mg Lithiumaluminiumhydrid und rührt 2 Stunden unter Eiskühlung, 16 Stunden bei Raumtemperatur und I Stunde unter Rückfluß. Nach Abkühlen versetzt man mit gesättigter Kochsalzlösung, extrahiert mit Essigester, trocknet die organische Phase mit Natriumsulfat und dampft das Lösungsmittel ein. Der Rückstand wird an Kieselgel mit Hexan und Essigester chromatografiert, man isoliert 42 mg 11 β-Fluor-gonadiol (59 % d. Th.) als festen Schaum.

### Beispiel 2 11β-Methyl-gona-1,3,5(10)-trien-3,17-diol

### 2.1 11-β-Methyl-3-mesyloxy-estra-1,3,5(10)-17-on

28,4 g 11β-Methyl-estra-1,3,5(10)-3-ol-17-on (100 mmol, Gantchev, J.Med.Chem 1994, 4164) werden wie in Beispiel 1.2 beschrieben in das Mesylat überführt, Ausbeute 33,5 g (92 % d. Th.) als festen Schaum.

### 2.2 11β-Methyl-3-mesyloxy-17-oximinoestra-1,3,5(10)-trien

Die Herstellung des Oxims erfolgt wie in Beispiel 1.3 beschrieben in 89 % Ausbeute (34,0 g), Fp.

### 2.3 11β-Methyl-3-mesyloxy-13,17-seco-estra-1,3,5(10),13(18)-tetraen-17-nitril

Das Oxim wird wie in Beispiel 1.4 beschrieben in die Secoverbindung umgewandelt, sie fällt als fester Schaum in einer Ausbeute von 9,1 g (28 % d. Th.) an.

### 2.4 13(18)-Epoxy-11β-methyl-3-mesyloxy-13,17-seco-estra-1,3,5(10)-trien-17-nitril

Die Epoxidierung wird wie in Beispiel 1.5 beschrieben durchgeführt und liefert 6,7 g Epoxid (71 % d. Th.) als farbloses Öl.

### 2.5. 11β-Methyl-3-mesyloxy-gona-1,3,5(10)-trien-17-on

Durch Cyclisierung mit Bortrifluoridetherat erhält man wie in Beispiel 1.6 beschrieben das Gonadiol-Derivat in einer Ausbeute von 14 % d. Th. (780 mg).

### 2.6 11β-Methyl-gona-1,3,5(10)-trien-3,17-diol

Die Reduktion der Carbonylgruppe und Abspaltung der Schutzgruppe mit Lithiumalanat zum Endprodukt (wie Beispiel 1.7) gelingt in 48 % Ausbeute (290 mg).

### Beispiel 3 11β-Ethyl-gona-1,3,5(10)-trien-3,17-diol

Wie in Beispiel 1 beschrieben wird das 11β-Ethyl-18-nor-estradiol aus 11β-Ethyl-estra-1,3,5(10)-trien-3-ol-17-on (Pomper, J.Med.Chem. 1990, 3143) in einer Gesamtausbeute von 1,3 % hergestellt, Fp.

### Beispiel 4 11β-Phenyl-gona-1,3,5(10)-trien-3,17-diol

In gleicher Weise wird aus 11β-Phenyl-estra-1,3,5(10)-trien-3-ol-17-on (Tedesco, J.Org.Chem. 1995, 5316) die entsprechende 18-Nor-Verbindung hergestellt, Gesamtausbeute 0,7 %, Fp.

### Beispiel 5 7α-Fluor-gona-1,3,5(10)-trien-3,17-diol

### 5.1 3-Benzyloxy-7β-hydroxy-estra-1,3,5(10)-trien-17-on

Zu einer Suspension von 20 g (67,74 mmol) 3,7β-Dlhydroxy-estra-1,3,5(10)-trien-17-on, 3,55 g (148,23 mmol) Lithiumhydroxid in 700 ml trockenem Dimethylformamid gibt man 12,83 g (75 mmol) Benzylbromid und rührt unter einer Argonatmosphäre 30 Minuten bei 100 °C. Zur Aufarbeitung gießt man die Reaktionslösung in weinsaures Eiswasser, saugt das ausgefallene Produkt ab und trocknet an der Luft. Das Rohprodukt wird in Dichlormethan aufgenommen, die organische Phase mit Wasser gewaschen und über Natriumsulfat getrocknet. Nach Abziehen des Lösungsmittels im Vakuum wird an Kieselgel chromatografiert (Dichlormethan-Essigester, Gradient bis 3:2), Ausbeute 22,81 g (87 %).

### 5.2 3-Benzyloxy-7α-fluor-estra-1,3,5(10)-trien-17-on

Man legt 1,50 g (4 mmol) 7β-Alkohol mit 1,52 g (10 mmol) DBU in 30 ml trockenem Toluol vor, kühlt unter Rühren und Feuchtigkeitsausschluß (Argonatmosphäre) auf 0 °C ab und versetzt tropfenweise mit 1,51 g (5 mmol) Perfluorbutansulfonsäurefluorid in 10 ml Toluol. Anschließend entfernt man das Eisbad und läßt noch 5 Stunden bei Raumtemperatur rühren. Zur Aufarbeitung verdünnt man mit Essigester (150 ml), wäscht die organische Phase zunächst mit verdünnter Salzsäure, dann mit Natronlauge und abschließend mit Wasser/Sole. Man läßt über Natriumsulfat trocknen, engt im Vakuum ein und zeigt den Rückstand mit Essigester aus. Nach erneutem Einengen wird der Rückstand an Kieselgel chromatografiert (Toluol-Essigester, Gradient bis 95:5), Ausbeute 0,35 g (23 %).

### 5.3 3-Benzyloxy-7α-fluor-gona-1,3,5-trien-17-on

Aus 37,8 g 3-Benzyloxy-7α-fluor-estra-1,3,5-trien-17-on erhält man wie in Beispiel 1.2 bis 1.6 beschrieben 3-Benzyloxy-7α-fluor-gona-1,3,5(10)-trien-17-on in einer Ausbeute von 1,03 g (2,8 % d. Th.) als festen Schaum.

### 5.4 7α-Fluor-18-nor-1,3,5(10)-trien3-ol-17-on

Man versetzt 0,30 g (0,79 mmol) benzyliertes 7α-Fluor-18-nor-estron mit einer Mischung aus 3 ml Thioanisol und 2 ml Trifluoressigsäure und läßt über Nacht bei Raumtemperatur unter Schutzgas und Feuchtigkeitsausschluß stehen. Zur Aufarbeitung rührt man in Eis/Kalilauge ein, extrahiert mit Essigester, wäscht die organische Phase neutral und trocknet über Natriumsulfat. Das Rohprodukt wird an Kieselgel chromatografiert (Toluol-Essigester, Gradient bis 4:1), Ausbeute 0,200 g (87 %).

### 5.5 7α-Fluor-8-nor-estra-1,3,5(10)-trien-3,17β-diol

0,22 g (0,76 mmol) 7α-Fluor-18-nor-estron werden in einer Mischung aus 3 ml Dichlormethan und 9 ml Methanol gelöst und unter Schutzgasatmosphäre bei 0 °C mit 0,35 g Natriumborhydrid portionsweise versetzt. Anschließend rührt man noch 30 Minuten bei Raumtemperatur, verdünnt mit Dichlormethan (150 ml) und arbeitet weinsauer auf. Die organische Phase wird nach Waschen mit Wasser über Natriumsulfat getrocknet. Das Rohprodukt wird nach Abziehen des Lösungsmittels an Kieselgel chromatografiert (Toluol-Essigester, Gradient bis 1:1), Ausbeute 0,22 g (quant.) als festen Schaum.

### Beispiel 6 7α-Methyl-gona-1,3,5(10)-trien-3,17β-diol

Wie in Beispiel 1 beschrieben wird 7α-Methyl-estra-1,3,5(10)-trien-3-ol-17-on (Ali et. al., J.Med. Chem. 1993, 264) in einer Ausbeute von 1,8 % in das Endprodukt überführt. Fp.

### Beispiel 7 7α-Phenyl-gona-1,3,5(10)-trien-3,17β-diol

### 7.1. 7α-Phenyl-estr-4-en-3,17-dion

Man suspendiert 5,76 g Magnesiumspäne (237 mmol) in 60 ml wasserfreiem THF und gibt bis zum Anspringen der Reaktion langsam, dann rascher insgesamt 36,1 g Brombenzol (230 mmol) gelöst in 100 ml wasserfreiem THF zu und erwärmt 1 Stunde auf 80 °C. Die Mischung von 24 g Kupfer-I-iodid (120 mmol) und 43 g Lithiumbromid (480 mmol) in 115 ml wasserfreiem THL erwärmt sich auf etwa 50 °C, dann gibt man 40 ml DMPU zu und rührt 30 Minuten. Die Lösung von Phenylmagnesiumiodid wird auf -60 °C abgekühlt und langsam mit der Lösung des Kupfersalzes versetzt. Danach wird 15 Minuten bei -30 °C gehalten, wieder auf-65 °C abgekühlt und mit einer Lösung von 12,4 g Estra-4,6-dien-3,17-dion (46 mmol) in 115 ml wasserfreiem THF versetzt. Man läßt die Mischung nach der letzten Zugabe innerhalb von 45 Minuten auf -5 °C erwärmen, kühlt wieder auf -40 °C ab und fügt 15 ml Trimethylchlorsilan (115 mmol) zu. Während der nächsten 45 Minuten steigt die Innentemperatur auf -18 °C an, man versetzt mit 15 ml Eisessig, enternt das Kühlbad und rührt eine weitere Stunde. Anschließend wird mit Essigester verdünnt, wäscht mit halbgesättigter Ammoniumchloridlösung, gesättigter Bicarbonatlösung und Kochsalzlösung aus, trocknet die organische Phase mit Natirumsulfat und dampft das Lösungs-mittel ein. Der Rückstand wird an Kieselgel mit Hexan, Methylenchlorid und Essigester chromato-graphiert. Die Ausbeute an 7α-Phenyl-estr-4-en-3,17-dion beträgt 8,6 g (54 % d. Th.).

### 7.2. 7α-Phenyl-gona-1,3,5(10)-trien-3,17β-diol

Die Synthesesequenz wird wie in Beispiel 1.1 bis 1.7 beschrieben durchgeführt. Die Ausbeute über allen Stufen beträgt 2,3 %.

### Beispiel 8 7α-Methyl-gona-1,3,5(10)-trien-1,3,17-triol

### 8.1 7α-Methyl-estra-1,3,5(10)-trien-1,3-diol-17-on

Die Lösung von 38,4 g Diacetoxy-7α-methyl-1,3,5(10)-estra-1,3,5(10)-trien-17-on (100 mmol; Sauer, Chem.Ber. 1982, 459) löst man in 300 ml wasserfreiem Ethanol, gibt 30 ml 4n-NaOH zu und erwärmt 30 Minuten auf 60 °C. Danach wird das Lösungsmittel weitgehend abdestilliert, der Rückstand in Essigester aufgenommen und mit Wasser neutral gewaschen. Die organische Phase trocknet man mit Natriumsulfat und dampft ein. Der Rückstand wird an Kieselgel mit Benzin-Essigester chromatografiert und aus Essigester/Diisopropylether kristallisiert, Ausbeute 24 g (80 % d. Th.), Fp 228-229 °C, [α_{-D}] = +209 ° (0,5 % in Pyridin).

### 8.2 7α-Methyl-gona-1,3,5(10)-trien-1,3,17-triol

Die Synthesesequenz wird wie in Beispiel 1.2 bis 1.7 beschrieben durchgeführt. Die Ausbeute über alle Stufen beträgt 4,5 %.

### Beispiel 9 2-Fluor-gona-1,3,5(10)-trien-3,17β-diol

### 9.1 2-Fluor-3-methoxy-gona-1,3,5(10)-trien-17β-ol

Ausgehend von 30,2 g 2-Fluor-3-methoxy-estra-1,3,5(10)-trien-17-on (100 mmol, Diorazio, J.C.S.Perkin I **1992,** 421) wird die Synthese der 18-Nor-Verbindung wie in Beispiel 1.3 bis 1.7 beschrieben durchgeführt, Ausbeute 5,3 %

### 9.2 2-Fluor-gona-1,3,5(10)-trien-3,17-diol

Die Lösung von 0,53 g 2-Fluor-3-methoxy-gona-1,3,5(10)-trien-17β-ol in 50 ml Toluol wird mit 5 ml 20 %iger Lösung von DIBAH in Toluol versetzt und 2 Stunden auf 80 °C erwärmt. Nach dem Abkühlen fügt man tropfenweise Wasser zu, bis die Reaktion abgeklungen ist, verteilt dann zwischen Wasser und Essigester, wäscht die Essigesterphase mit konzentrierter Kochsalzlösung und trocknet mit Natriumsulfat. Nach Abdampfen bleibt ein Rückstand, der an Kieselgel mit Hexan-Essigester chromatografiert wird, die Ausbeute beträgt 0,32 g (63 % d. Th.).

### Beispiel 10 11β,17α-Dimethylgona-1,3,5(10)-trien-3,17β-diol

### 10.1 5,6α-Epoxy-3,3-[1,2-ethandiylbis(oxy)]-11-methylen-5α-gonan-17-on

1,4 g (4,45 mmol) 3,3-[1,2-Ethandiylbis(oxy)-11,methylengon-5-en-17-on (Herstellung siehe DE 19S3S851, Beispiel 2c) werden in 20 ml Dichlormethan gelöst. Man versetzt mit 1,5 ml gesättigter wäßriger Natriumhydrogencarbonatlösung, kühlt dann auf 0°C und addiert bei dieser Temperatur 490 mg (18,3 mmol) 2,2,2-Trifluor-1-(3-nitrophenyl)ethanon sowie 1,9 ml Wasserstoffperoxid (30%ige wäßrige Lösung, 18,6 mmol). Danach wird 100 Stunden bei 25°C gerührt. Anschließend werden bei leichter Kühlung 9 ml gesättigte Natriumthiosulfatlösung zu dem Reaktionsgemisch addiert. Man extrahiert mit Dichlormethan. Die organische Phase wird mit 5%iger Natriumhydroxidlösung sowie gesättigter Natriumchloridlösung gewaschen und über Natriumsulfat getrocknet. Säulenchromatographie des Rohprodukts an Kieselgel mit einem Gemisch aus Hexan/Ethylacetat ergibt 1 g (68%) 10.1.
¹H-NMR (CDCl₃): d= 4,97 (1H), 4,69 (1H), 3,85-4,08 (1H), 3,02 (1H), 2,66 (1H) ppm.

### 10.2 3,3-[1,2-Ethandiylbis(oxy)]-11-methylen-5α-gonan-5,17β-diol

Zu einer Suspension von 990 mg (26 mmol) Lithiumaluminiumhydrid in 20 ml Tetrahydrofuran wird bei 0°C eine Lösung von 1 g (3,03 mmol) der unter a) beschriebenen Verbindung in 10 ml Tetrahydrofuran addiert. Man rührt eine Stunde bei 0°C nach und addiert dann vorsichtig 20 ml gesättigte Ammoniumchloridlösung. Der sich bildende Niederschlag wird abfiltriert. Das Filtrat wird mit Ethylacetat verdünnt. Man wäscht dann die organische Phase mit gesättigter Natriumchloridlösung und trocknet über Natriumsulfat. Säulenchromatographie des Rohprodukts an Kieselgel mit einem Gemisch aus Hexan/Ethylacetat ergibt 980 mg (90%) 10.2. ¹H-NMR (CDCl₃): δ= 4,88 (1H), 4,54 (1H), 3,90-4,06 (4H), 3,84 (1H) ppm.

### 10.3 3,3-[1,2-Ethandiylbis(oxy)]-11β-methyl-5α-gonan-5,17β-diol

Eine Lösung von 980 mg (2,93 mmol) der unter b) beschriebenen Verbindung in 80 ml Ethanol wird mit 90 mg Palladium/Kohle (10%) versetzt. Man hydriert dann unter 1 Atmosphäre Wasserstoff (Reaktionszeit ca. 20 min). Anschließend wird über Celite/Kieselgel filtriert und im Vakuum eingeengt. Das erhaltene Rohprodukt (986 mg (100%) wird ohne Aufreinigung in die Folgestufe eingesetzt.
¹H-NMR (CDCl₃): δ= 3,89-4,06 (4H), 3,86 (1H), 3,73 (1H), 0,86 (3H) ppm.

### 10.4 3,3-[1,2-Ethandiylbis(oxy)]-5-hydrox11β-methyl-5α-gonan-17-on

Zu 5,9 ml Pyridin in 30 ml Dichlormethan werden bei 0°C 1,76 g (17,6 mmol) Chromtrioxid addiert. Man läßt 15 Minuten bei 0°C nachrühren und addiert dann eine Lösung von 986 mg (2,93 mmol) der unter c) beschriebenen Verbindung in 10 ml Dichlormethan. Anschließend läßt man eine Stunde bei 0°C nachrühren und wäscht danach zweimal mit 5%iger wäßriger Natriumhydroxidlösung und einmal mit gesättigter wäßriger Natriumchloridlösung. Man trocknet über Natriumsulfat und reinigt das erhaltene Rohprodukt durch Säulenchromatographie an Kieselgel mit einem Gemisch aus Hexan/Ethylacetat. Es werden 830 mg (85%) 10.4 erhalten.
¹H-NMR (CDCl₃); δ= 3,92-4,05 (4H), 3,89 (1H), 2,38 (1H), 0,85 (3H) ppm.

### 10.5 11β,17α-Dimethyl-3,3-[1,2-ethandiylbis(oxy)]-5α-gonan-5,17β-diol (A) und 11β,17β-Dimethyl-3,3-[1,2-ethandiylbis(oxy)]-5α-gonan-5,17α-diol (B)

Eine Lösung von 260 mg (0,78 mmol) der unter c) beschrieben Verbindung in einem Gemisch aus 5 ml Tetrahydrofuran und 5 ml Diethylether wird bei 0°C zu 4,7 ml einer 1,6 molaren Lösung von Methyllithium in Diethylether addiert. Man läßt eine Stunde bei 0°C nachrühren und gießt dann das Reaktionsgemisch auf gesättigte wäßrige Ammoniumchloridlösung. Man extrahiert mit Ethylacetat, wäscht die organische Phase mit gesättigter Natriumchloridlösung und trocknet über Natriumsulfat. Das erhaltenen Rohprodukt wird durch Säulenchromatographie an Kieselgel mit einem Gemisch aus Hexan/Ethylacetat gereinigt. Es werden 166 mg (61%) der Verbindung A und 60 mg (22%) der Verbindung B erhalten.
¹H-NMR (CDCl₃):
Verbindung A: δ= 3,90-4,02 (3H), 2,08 (1H), 1,12 (3H), 0,85 (3H) ppm.
Verbindung B: δ= 3,90-4,03 (3H), 2,12 (1H), 1,28 (3H), 0,85 (3H) ppm.

### 10.6 11β,17α-Dimethyl-17β-hydroxy-gon-4-en-3-on

Zu einer Lösung von 166 mg (0,47 mmol) der unter e) beschriebenen Verbindung A in 10 ml Aceton werden 0,4 ml 4 normale Salzsäure addiert. Man läßt eine Stunde bei 25°C nachrühren und gießt dann das Reaktionsgemisch auf gesättigte wäßrige Natriumhydrogencarbonatlösung. Anschließend wird mit Dichlormethan extrahiert. Die organische Phase wird mit gesättigter Natriumchloridlösung gewaschen und über Natriumsulfat getrocknet. Das erhaltenen Rohprodukt wird durch Säulenchromatographie an Kieselgel mit einem Gemisch aus Hexan/Ethylacetat gereinigt. Es werden 119 mg (87%) 10.6 erhalten.
¹H-NMR (CDCl₃): δ= 5,83 (1H), 1,13 (3H), 0,97 (3H) ppm.

### 10.7 11β,17α-Dimethylgona-1,3,5(10)-trien-3,17β-diol

Aus 0,77 ml einer 1,6 molaren Lösung von n-Butyllithium in Hexan (1,23 mmol) und 0,18 ml (1,28 mmol) Diisopropylamin wird in 10 ml Tetrahydrofuran bei 0°C Lithiumdiisopropylamid (LDA) hergestellt. Anschließend kühlt man auf-78°C und addiert eine Lösung von 119 mg (0,41 mmol) der unter 10.6 beschriebenen Verbindung in 8 ml Tetrahydrofuran. Es wird eine Stunde bei -78°C nachgerührt. Danach werden 0,16 ml (0,42 mmol) Trimethylchlorsilan addiert. Man läßt auf 0°C kommen und rührt weitere 30 Minuten nach. Anschließend wird das Reaktionsgemisch auf gesättigte wäßrige Natriumhydrogencarbonatlösung gegossen. Man extrahiert mit Ethylacetat, wäscht die organische Phase mit gesättigter wäßriger Natriumchloridlösung, trocknet über Natriumsulfat und engt im Vakuum ein. Der erhaltene rohe Silylenolether (148 mg) wird in 6 ml Acetonitril gelöst. Man addiert 102 mg (0,45 mmol) Palladium(II)acetat und läßt 2,5 Stunden bei 25°C nachrühren. Danach wird das Reaktionsgemisch über Celite filtriert und das Filtrat im Vakuum eingeengt. Das erhaltenen Rohprodukt wird durch Säulenchromatographie an Kieselgel mit einem Gemisch aus Hexan/Ethylacetat gereinigt. Es werden 61 mg (52%) der Titelverbindung erhalten.
¹H-NMR (CDCl₃): δ= 7,04 (1H), 6,58 (1H), 6,47 (1H), 2,60-2,75 (3H), 2,40 (1H), 1,14 (3H), 0,72 (3H) ppm.

### Beispiel 11 11β,17β-Dimethylgona-1,3,5(10)-trien-3,17α-diol

### 11.1 11β,17β-Dimethyl-17α-hydroxy-gon-4-en-3-on

Analog zu Beispiel 10.6 werden aus 60 mg (0,17 mmol) der unter 10.5 beschrieben Verbindung B durch Umsetzung mit 4 normaler Salzsäure in Aceton nach Säulenchromatographie 40 mg (81%) 11.1 erhalten.
¹H-NMR (CDCl₃): δ= 5,83 (1H), 1,29 (3H), 0,98 (3H) ppm.

### 11.2 11β,17β-Dimethylgona-1,3,5(10)-trien-3,17α-diol

Analog zu Beispiel 10.7 werden aus 40 mg (0,14 mmol) der unter 11.1 beschriebenen Verbindung 23 mg (57%) 11.2 erhalten.
¹H-NMR (CDCl₃): δ= 7,09 (1H), 6,62 (1H), 6,50 (1H), 2,70-2,85 (3H), 2,48 (1H), 1,29 (3H), 0,78 (3H) ppm.

### Beispiel 12 11β-Methyl-17α-(1-propinyl)-gona-1,3,5(10)-trien-3,17β-diol

| | | |
|---|---|---|
| Substratmenge: | 6,0 mg 17β-Hydroxy-11β-methyl-17α-(1-propinyl)-gon-4-en-3-on | |
| | Die Herstellung der Titelverbindung mit dem Stamm *Bacillus lentus* ATCC 13805 erfolgt in einem 20 ml Fermentationskolben. | |
| Vorkultur: | 100 ml steriles Medium bestehend aus 0,5% Glucose, 0,5% Hefeextrakt, 0,1% Pepton und 0,2% Coom steep liquor (pH 7,5) wird mit einer Schrägrörchenkultur des Stammes *Bacillus lentus* ATCC 13805 beimpft und 24 Stunden bei 28°C und 165 upm auf einem Rotationsschüttler inkubiert. | |
| Hauptkultur: | Für die Biotransformation werden 3 x 20 ml der Vorkultur in je einen sterilen 100 ml Schüttelkolben überführt. Zur gleichen Zeit wird das Substrat 17β-Hydroxy-11β-methyl-17α-(1-propinyl)-gon-4-en-3-on in einem organischen Lösungsmittel gelöst | |
| | und zugegeben. Die Konzentration in der Kulturbrühe beträgt 100 mg/l. Als Lösungsmittel wird bevorzugt Ethanol eingesetzt, es können aber auch andere mit Wasser mischbare Lösungsmittel wie z. B. Dimethylformamid verwendet werden. Die Inkubation wird bei 28°C und 165 upm auf einem Rotationsschüttler durchgeführt. Die Kontrolle der Umsetzung erfolgt unter Einsatz von Methoden wie Dünnschichtchromatographie oder bevorzugt HPLC. Nach 28 Stunden ist die Reaktion beendet. Die Kulturbrühe der drei Schüttelkolben wird 1 x mit je 1 Vol. Methylisobutylketon extrahiert, die organischen Phasen werden vereinigt und unter Vakuum zur Trockene eingeengt. | |
| Isolierung: | Die Isolierung und Reinigung des Reaktionsprodukts 11β-Methyl-17α-(1-propinyl)-gona-1,3,5(10)-trien-3,17β-diol erfolgte (wegen der geringen Menge) über HPLC unter folgenden Bedingungen: | |
| | Säule: | Semi-Präp-Säule der Fa. Phenomenex (LUNA 5µ, SILICA (2), 250 x 10 mm), |
| | Fließmittel: | Hexan/Dioxan 75/25 |
| | Flußrate: | 5 ml/min |
| Ausbeute: | 42% 11β-Methyl-17α-(1-propinyl)-gona-1,3,5(10)-trien-3,17β-diol | |

### Beispiel 13 11-Methylen-17α-(1-propinyl)-gona-1,3,5(10)-trien-3,17β-diol

In Analogie zu Beispiel 12 wird aus 20 mg 17β-Hydroxy-11-methylen-17α-(1-propinyl)-gon-4-en-3-on die Titelverbindung in 90% Ausbeute erhalten.

### Beispiel 14 17α-(1-propinyl)-gona-1,3,5(10)-trien-3,17β-diol

In Analogie zu Beispiel 12 wird aus 6 mg 17β-Hydroxy-17α-(1-propinyl)-gon-4-en-3-on die Titelverbindung in 40% Ausbeute erhalten.

**Tabelle 1**

| **Estrogen** | **Struktur** | **hER α RBA*** | **hER β RBA*** | **ERβ/ ERα** | **Rat uterus ER(RBA)** | **Rat prost. ER(RBA)** | **prost.ER/ uterusER** |
|---|---|---|---|---|---|---|---|
| **Estradiol** | 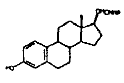 | **100** | **100** | **1** | **100** | **100** | **1** |
| **Estron** | 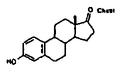 | **60** | **37** | **0.6** | **3** | **2** | **0.8** |
| **17α-Estradiol** | 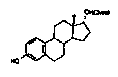 | **58** | **11** | **0.2** | **2.4** | **1.3** | **0.5** |
| **Estriol** | 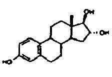 | **14** | **21** | **1.5** | **4** | **20** | **5** |
| **5-Androsten -diol** | 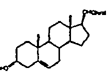 | **6** | **17** | **3** | **0.1** | **5** | **50** |
| **Genistein** | 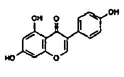 | **5** | **36** | **7** | **0.1** | **10** | **100** |
| **Coumestrol** | 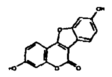 | **94** | **185** | **2** | **1.3** | **24** | **18** |

| | | | | | | | |
|---|---|---|---|---|---|---|---|
| *: zitiert aus : Kuiper et al. (1996), Endocrinology 138: 863-870 | | | | | | | |

**Tabelle 2**

| **Verbindung** | **ER Prostata RBA** | **ER Uterus RBA** |
|---|---|---|
| A | 37 | 3 |
| B | 45 | 26 |
| C | 77 | 4 |
| D | 10 | 0,4 |
| E | 111 | 20 |

## Patentansprüche

1. Gonatrien-derivate nach Anspruch 1, nämlich
11β-Fluor-gona-1,3,5(10)-trien-3,17-diol
11β-Chlor-gona-1,3,5(10)-trien-3,17-diol
11β-Methyl-gona-1,3,5(10)-trien-3,17-diol
11β-Ethyl-gona-1,3,5(10)-trien-3,17-diol
11β-Phenyl-gona-1,3,5(10)-trien-3,17-diol
7α-Fluor-gona-1,3,5(10)-trien-3,17-diol
7α-Methyl-gona-1,3,5(10)-trien-3,17β-diol
7α-Phenyl-gona-1,3,5(10)-trien-3,17β-diol
7α-Methyl-gona-1,3,5(10)-trien-3,17-diol
7β-Ethyl-gona-1,3,5(10)-trien-3,17β-diol
7β-Ethyl-gona-1,3,5(10)-trien-3,17α-diol
7β-Ethyl-13α-H-gona-1,3,5(10)-trien-3,17α-diol
7β-Fluor-gona-1,3,5(10)-trien-3,17-diol
7β-Methyl-gona-1,3,5(10)-trien-3,17β-diol
7β-Phenyl-gona-1,3,5(10)-trien-3,17β-diol
7β-Methyl-gona-1,3,5(10)-trien-3,17-diol
2-Fluor-gona-1,3,5(10)-trien-3,17β-diol
17α-(1-Propinyl)-gona-1,3,5(10)-trien-3,17β-diol
11-Methylen-17α-(1-propinyl)-gona-1,3,5(10)-trien-3,17β-diol
11β-Methyl-17α-(1-propinyl)-gona-1,3,5(10)-trien-3,17β-diol
11β,17α-Dimethyl-gona-1,3,5(10)-trien-3,17β-diol
11β,17β-Dimethyl-gona-1,3,5(10)-trien-3,17α-diol
18-Nor-estriol
13α-H-18-Nor-estradiol
11β-Fluor-gona-1,3,5(10)-trien-1,3,17-triol
11β-Chlor-gona-1,3,5(10)-trien-1,3,17-triol
11β-Methyl-gona-1,3,5(10)-trien-1,3,17-triol
11β-Ethyl-gona-1,3,5(10)-trien-1,3,17-triol
11β-Phenyl-gona-1,3,5(10)-trien-1,3,17-triol
7α-Fluor-gona-1,3,5(10)-trien-1,3,17-triol
7α-Methyl-gona-1,3,5(10)-trien-1,3,17-triol
7α-Phenyl-gona-1,3,5(10)-trien-1,3,17-triol
7α-Methyl-gona-1,3,5(10)-trien-1,3,17-triol
7β-Fluor-gona-1,3,5(10)-trien-1,3,17-triol
7β-Methyl-gona-1,3,5(10)-trien-1,3,17-triol
7β-Phenyl-gona-1,3,5(110)-trien-1,3,17-triol
7β-Methyl-gona-1,3,5(10)-trien-1,3,17-triol
2-Fluor-gona-1,3,5(10)-trien-1,3,17-triol
17α-(1-Propinyl)-gona-1,3,5(10)-trien-1,3,17-triol
11-Methylen-17α-(1-propinyl)-gona-1,3,5(10)-trien-1,3,17-triol
11β-Methyl-17α-(1-propinyl)-gona-1,3,5(10)-trien-1,3,17-triol
11β,17α-Dimethyl-gona-1,3,5(10)-trien-1,3,17-triol
11β,17β-Dimethyl-gona-1,3,5(10)-trien-1,3,17-triol.

2. Verwendung von Gona-1,3,5(10)-trien-derivaten der allgemeinen Formel I'a 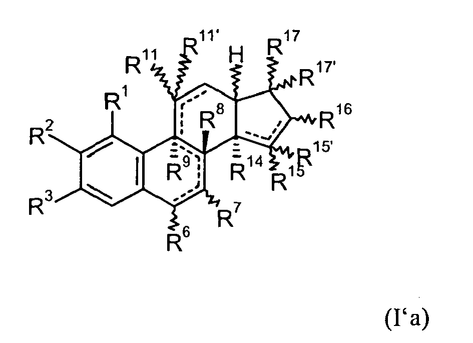 worin
R¹ ein Halogenatom, ein Rest R¹⁸- oder R¹⁸-O-, wobei R¹⁸ ein Wasserstoffatom oder einen gerad- oder verzweigtkettigen, gesättigten oder ungesättigten Kohlenwasserstoffrest mit bis zu 6 Kohlenstoffatomen, eine Trifluormethylgruppe, bedeutet;
R² ein Halogenatom;
ein Rest R¹⁸- oder R¹⁸-O-, wobei R¹⁸ die unter R¹ angegebene Bedeutung hat; eine Gruppe R¹⁹SO₂-O-, worin R¹⁹ eine R²⁰R²¹N - Gruppe ist, wobei R²⁰ und R²¹ unabhängig voneinander ein Wasserstoffatom, einen C₁ - C₅ - Alkylrest wobei Alkylrest auch teilweise oder vollständig fluoriert oder substituiert sind durch 1-5 Halogenatome, Hydroxygruppen oder C1-C4-Alkoxygruppen, eine Gruppe C(O)R²², worin R²² einen gerad- oder verzweigtkettigen Kohlenwasserstoffrest mit bis zu 12 Kohlenstoffatom, der außerdem bis zu drei Doppel- und/oder Dreifachbindungen enthalten kann, einen C₃ - C₇ - Cycloalkylrest, einen Arylrest mit Aryl, welches immer auch einen Heteroarylrest mit einschließt, einen Phenyl-, 1- oder 2-Naphthylrest, oder eine Kombination aus diesen Strukturmerkmalen darstellt, oder, zusammen mit dem N-Atom, einen Polymethyleniminorest mit 4 bis 6 C-Atomen oder einen Morpholinorest, bedeuten;
R³ eine Gruppe R¹⁸-O-, R¹⁹SO₂-O- oder -O-C(O)R²², mit R¹⁸, R¹⁹ und R²² jeweils in der unter R¹ bzw. R² angegebenen Bedeutung, wobei für R¹⁸ zusätzlich ein Aryl-, Heteroaryl- oder Aralkylrest wobei es sich bei einem Aralkylrest um einen Rest handelt, der im Ring bis 14, bevorzugt 6 bis 10, C-Atome und in der Alkylkette 1 bis 8, bevorzugt 1 bis 4, C-Atome enthält, und aliphatische, gerad- oder verzweigtkettige, gesättigte oder ungesättigte C₁-C₁₄-Mono- oder Polycarbonsäure oder eine aromatische Carbonsäure oder eine α- oder β-Aminosäure stehen kann;;
R⁶ und R⁷ unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R¹ bzw. R² angegebenen Bedeutung, eine gerad- oder verzweigtkettige, gesättigte oder ungesättigte, teilweise oder vollständig halogenierte Alkylgruppe mit bis zu 10 Kohlenstoffatomen oder ein gegebenenfalls substituierter Aryl-, Heteroaryl- oder Aralkylrest,
R⁸ und R⁹ unabhängig voneinander ein Wasserstoffatom, einen gerad- oder verzweigtkettigen, gesättigten oder ungesättigten, gegebenenfalls teilweise oder vollständig halogenierten Kohlenwasserstoffrest mit bis zu 10 Kohlenstoffatomen, eine Gruppe -X- R¹⁸, worin X ein Sauerstoff- oder Schwefelatom ist und R¹⁸ die unter R¹ angegebene Bedeutung hat, ein Halogenatom, eine Cyano-oder Rhodanogruppe,
R¹¹ ein Wasserstoffatom, ein Halogenatom, eine Gruppe R¹⁹SO₂-O- oder - R²², mit R¹⁸, R¹⁹ und R²² jeweils in der unter R¹ bzw. R² angegebenen Bedeutung, eine Gruppe -X- R¹⁸, worin X ein Sauerstoff- oder Schwefelatom ist und R¹⁸ die unter R¹ angegebene Bedeutung hat, eine Nitrooxygruppe, einen gerad- oder verzweigtkettigen, gesättigten oder ungesättigten, teilweise oder vollständig halogenierten Kohlenwasserstoffrest mit bis zu 10 Kohlenstoffatomen oder ein gegebenenfalls substituierter Aryl-, Heteroaryl- oder Aralkylrest, sowie
R^{11'} ein Wasserstoffatom oder
R¹¹ und R^{11'} gemeinsam eine Methylengruppe
R¹⁴ ein α-ständiges Wasserstoffatom oder eine gerad- oder verzweigtkettige Alkyl-, Alkenyl- oder Alkinylgruppe mit bis zu 3 Kohlenstoffatomen und
R¹⁵ ein Wasserstoffatom
oder
R¹⁴undR15 zusammen eine gegebenenfalls ein- oder zweifach halogenierte Methylengruppe;
R^{15'} und R¹⁶ unabhängig voneinander ein Wasserstoffatom, ein Halogenatom, eine Gruppe R¹⁸-O-, R¹⁹SO₂-O- oder -R²², mit R¹⁸, R¹⁹ und R²² jeweils in der unter R¹ bzw. R² angegebenen Bedeutung;
R¹⁷ und R^{17'} ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder-O-C(O)R²², mit R¹⁸, R¹⁹ und R²² jeweils in der unter R¹ bzw. R² angegebenen Bedeutung; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R¹ bzw. R² angegebenen Bedeutung und bei R¹⁸-O- mit R¹⁸ zusätzlich eine aliphatische, gerad- oder verzweigtkettige, gesättigte oder ungesättigte C₁-C₁₄-Mono- oder Polycarbonsäure oder eine aromatische Carbonsäure oder eine α- oder β-Aminosäure stehen kann; oder
R¹⁷ und R^{17'} gemeinsam eine Gruppe =CR²³R²⁴, worin R²³ und R²⁴ unabhängig voneinander ein Wasserstoffatom und ein Halogenatom darstellen, oder ein Sauerstoffatom;
bedeuten und
in den Positionen 6, 7; 7, 8; 9, 11; 11, 12; 14, 15 sowie 15, 16 eine oder mehrere Doppelbindungen oder 2 Doppelbindungen in den Positionen 6, 7 und 8, 9 vorhanden sein können
zur Herstellung eines Arzneimittels zur Behandlung von peri- und postmenopausalen Beschwerden.

3. Verwendung von Gonatrien-derivaten gemäß Anspruch 1 sowie 18-Nor-17β-estradiol zur Herstellung eines Arzneimittels nach Anspruch 2.

4. Verwendung der Gona-1,3,5(10)-trien-derivate der allgemeinen Formel I'a nach Anspruch 2 zur Herstellung eines Medikamentes zur Behandlung von peri- und postandropausalen Beschwerden.

5. Verwendung nach Anspruch 2 zur Vorbeugung gegen und Behandlung von Hitzewallungen, Schlafstörungen, Reizbarkeit, Stimmungsschwankungen, Inkontinenz, Vaginalatrophie und hormondefizienzbedingter Gemütserkrankungen.

6. Verwendung der Gona-1,3,5(10)-trien-derivate der allgemeinen Formel I'a nach Anspruch 2 zur Herstellung eines Medikamentes zur Vorbeugung und Behandlung von Erkrankungen im Urogenitaltrakt.

7. Verwendung der Gona-1,3,5(10)-trien-derivate der allgemeinen Formel I'a nach Anspruch 2 zur Herstellung eines Medikamentes zur Vorbeugung und Therapie von Magen- und Darmerkrankungen.

8. Verwendung nach Anspruch 7 zur Vorbeugung und Therapie von Ulcera und hemoragischen Diathesen im Magendarmtrakt.

9. Verwendung nach Anspruch 7 zur Vorbeugung und Therapie von Neoplasien.

10. Verwendung der Gona-1,3,5(10)-trien-derivate der allgemeinen Formel I'a nach Anspruch 2 zur Herstellung eines Medikamentes für die in-vitro Behandlung der männlichen Infertilität.

11. Verwendung der Gona-1,3,5(10)-trien-derivate der allgemeinen Formel I'a nach Anspruch 2 zur Herstellung eines Medikamentes für die in-vivo Behandlung der männlichen Infertilität.

12. Verwendung der Gona-1,3,5(10)-trien-derivate der allgemeinen Formel I'a nach Anspruch 2 zur Herstellung eines Medikamentes für die in-vitro Behandlung der weiblicheninfertilität.

13. Verwendung der Gona-1,3,5(10)-trien-derivate der allgemeinen Formel I'a nach Anspruch 2 zur Herstellung eines Medikamentes für die in-vivo Behandlung der weiblichen Infertilität.

14. Verwendung nach Anspruch 2 für die Hormonersatz-Therapie (HRT).

15. Verwendung der Gona-1,3,5(10)-trien-derivate der allgemeinen Formel I'a nach Anspruch 2 zur Herstellung eines Medikamentes für die Therapie von hormondefizienz bedingten Beschwerden bei chirurgisch, medikamentös oder anders bedingter ovarieller Dysfunktion.

16. Verwendung der Gona-1,3,5(10)-trien-derivate der allgemeinen Formel I'a nach Anspruch 2 zur Herstellung eines Medikamentes zur Prophylaxe und Therapie eines hormondefizienzbedingten Knochenmasseverlustes.

17. Verwendung nach Anspruch 16 zur Prophylaxe und Therapie der Osteoporose.

18. Verwendung von Gona-1,3,5(10)-trien-derivaten nach Anspruch 1 zur Herstellung eines Medikaments zur Vorbeugung gegen und Therapie von Herzkreislauferkrankungen.

19. Verwendung von Gona-1,3,5(10)-trien-derivaten nach Anspruch 1 zur Herstellung eines Medikaments zur Vorbeugung gegen und Behandlung von Gefäßerkrankungen.

20. Verwendung von Gona-1,3,5(10)-trien-derivaten nach Anspruch 18 zur Vorbeugung gegen und Behandlung von Atherosklerose.

21. Verwendung nach Anspruch 19 zur Vorbeugung und Behandlung neointimaler Hyperplasien.

22. Verwendung von Gona-1,3,5(10)-trien-derivaten nach Anspruch 1 zur Herstellung eines Medikaments zur Vorbeugung und Behandlung hormondefizienzbedingter neurodegenerativer Erkrankungen.

23. Verwendung von Gona-1,3,5(10)-trien-derivaten nach Anspruch 1 zur Herstellung eines Medikaments zur Vorbeugung und Behandlung der Alzheimerschen Krankheit sowie hormondefizienzbedingter Beeinträchtigung von Gedächtnis- und Lernfähigkeit.

24. Verwendung von Gona-1,3,5(10)-trien-derivaten nach Anspruch 1 zur Herstellung eines Medikaments zur Behandlung von entzündlichen Erkrankungen und Erkrankungen des Immunsystems.

25. Verwendung von Gona-1,3,5(10)-trien-derivaten nach Anspruch 1 zur Herstellung eines Medikaments zur Vorbeugung und Behandlung der benignen Prostatahyperplasie (BPH).

26. Phärmazeutische Zusammensetzungen, enthaltend mindestens eine Verbindung gemäß Anspruch 1 sowie einen pharmazeutisch verträglichen Träger.

## Claims

1. Gonatriene derivatives according to Claim 1, namely
11β-fluoro-gona-1,3,5(10)-triene-3,17-diol
11β-chloro-gona-1,3,5(10)-triene-3,17-diol
11β-methyl-gona-1,3,5(10)-triene-3,17-diol
11β-ethyl-gona-1,3,5(10)-triene-3,17-diol
11β-phenyl-gona-1,3,5(10)-triene-3,17-diol
7α-fluoro-gona-1,3,5(10)-triene-3,17-diol
7α-methyl-gona-1,3,5(10)-triene-3,17β-diol
7α-phenyl-gona-1,3,5(10)-triene-3,17β-diol
7α-methyl-gona-1,3,5(10)-triene-3,17-diol
7β-ethyl-gona-1,3,5(10)-trzene-3,17β-diol
7β-ethyl-gona-1,3,5(10)-triene-3,17α-diol
7β-ethyl-13α-H-gona-1,3,5(10)-triene-3,17α-diol
7β-fluoro-gona-1,3,5(10)-triene-3,17-diol
7β-methyl-gona-1,3,5(10)-triene-3,17β-diol
7β-phenyl-gona-1,3,5(10)-triene-3,17β-diol
7β-methyl-gona-1,3,5(10)-triene-3,17-diol
2-fluoro-gona-1,3,5(10)-triene-3,17β-diol
17α-(1-propinyl)-gona-1,3,5(10)-triene-3,17β-diol
11-methylene-17α-(1-propinyl)-gona-1,3,5(10)-triene-3,17β-diol
11β-methyl-17α-(1-propinyl)-gona-1,3,5-(10)-triene-3,17β-diol
11β,17α-dimethyl-gona-1,3,5(10)-triene-3,17β-diol
11β,17β-dimethyl-gona-1,3,5(10)-triene-3,17α-diol 18-nor-estriol
13α-H-18-nor-estradiol
11β-fluoro-gona-1,3,5(10)-triene-1,3,17-triol
11β-chloro-gona-1,3,5(10)-triene-1,3,17-triol
11β-methyl-gona-1,3,5(10)-triene-1,3,17-triol
11β-ethyl-gona-1,3,5(10)-triene-1,3,17-triol
11β-phenyl-gona-1,3,5(10)-triene-1,3,17-triol
7α-fluoro-gona-1,3,5(10)-triene-1,3,17-triol
7α-methyl-gona-1,3,5(10)-triene-1,3,17-triol
7α-phenyl-gona-1,3,5(10)-triene-1,3,17-triol
7α-methyl-gona-1,3,5(10)-triene-1,3,17-triol
7β-fluoro-gona-1,3,5(10)-triene-1,3,17-triol
7β-methyl-gona-1,3,5(10)-triene-1,3,17-triol
7β-phenyl-gona-1,3,5(10)-triene-1,3,17-triol
7β-methyl-gona-1,3,5(10)-triene-1,3,17-triol
2-fluoro-gona-1,3,5(10)-triene-1,3,17-triol
17α-(1-propinyl)-gona-1,3,5(10)-triene-1,3,17-triol
11-methylene-17α-(1-propinyl)-gona-1,3,5(10)-triene-1,3,17-triol
11β-methyl-17a-(1-propinyl)-gona-1,3,5(10)-triene-1,3,17-triol
11β,17α-dimethyl-gona-1,3,5(10)-triene-1,3,17-triol
11β,17β-dimethyl-gona-1,3,5(10)-triene-1,3,17-triol.

2. Use of gona-1,3,5(10)-triene derivatives of the general formula I'a 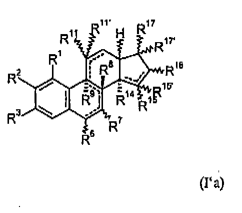 in which
R¹ means a halogen atom, an R¹⁸ or R¹⁸-O- radical, where R¹⁸ means a hydrogen atom or a straight-chain or branched-chain, saturated or unsaturated hydrocarbon radical with up to 6 carbon atoms, a trifluoromethyl group;
R² means a halogen atom;
an R¹⁸ or R¹⁸-O- radical, where R¹⁸ has the meaning that is indicated under R¹;
a group R¹⁹SO₂-O-, in which R¹⁹ is an R²⁰R²¹N group, where R²⁰ and R²¹, independently of one another, represent a hydrogen atom, a C₁-C₅ alkyl radical, where alkyl radical also are partially or completely fluorinated or substituted by 1-5 halogen atoms, hydroxyl groups or C₁-C₄ alkoxy groups, a group C(O)R²², in which R²² can contain a straight-chain or branched-chain hydrocarbon radical with up to 12 carbon atom, which in addition can contain up to three double bonds and/or triple bonds, a C₃-C₇ cycloalkyl radical, an aryl radical with aryl, which always also includes a heteroaryl radical, a phenyl or 1- or 2-naphthyl radical, or a combination of these structural features, or, together with the N atom, a polymethyleneimino radical with 4 to 6 C atoms or a morpholino radical;
R³ means a group R¹⁸-O-, R¹⁹SO₂-O- or O-C(O)R²², with R¹⁸, R¹⁹ and R²² in each case in the meaning that is indicated under R¹ and R², where in addition an aryl, heteroaryl or aralkyl radical can stand for R¹⁸, an aralkyl radical being a radical which contains 14, preferably 6 to 10, C atoms in the ring and 1 to 8, preferably 1 to 4, C atoms in the alkyl chain, and aliphatic, straight-chain or branched-chain, saturated or unsaturated C₁-C₁₉ mono- or polycarboxylic acid or an aromatic carboxylic acid or an α- or β-amino acid;
R⁶ and R⁷, independently of one another, mean a hydrogen atom, a halogen atom, a group R¹⁸-O-, R¹⁹SO₂-O or -R²², with R¹⁸, R¹⁹ and R²² in each case in the meaning that is indicated under R¹ and R², a straight-chain or branched-chain, saturated or unsaturated, partially or completely halogenated alkyl group with up to 10 carbon atoms or an optionally substituted aryl, heteroaryl or aralkyl radical,
R⁸ and R⁹, independently of one another, mean a hydrogen atom, a straight-chain or branched-chain, saturated or unsaturated, optionally partially or completely halogenated hydrocarbon radical with up to 10 carbon atoms, a group -X-R¹⁸, in which X is an oxygen or sulphur atom and R¹⁸ has the meaning that is indicated under R¹, a halogen atom, a cyano or rhodano group,
R¹¹ means a hydrogen atom, a halogen atom, a group R¹⁹SO₂-O- or -R²², with R¹⁸, R¹⁹ and R²² in each case in the meaning that is indicated under R¹ and R², a group -X-R¹⁸, in which X is an oxygen or sulphur atom and R¹⁸ has the meaning that is indicated under R¹, a nitrooxy group, a straight-chain or branched-chain, saturated or unsaturated, partially or completely halogenated hydrocarbon radical with up to 10 carbon atoms or an optionally substituted aryl, heteroaryl or aralkyl radical, and also
R^{11'} means a hydrogen atom or
R¹¹ and R^{11'} together mean a methylene group
R¹⁴ means a hydrogen atom in the α position or a straight-chain or branched-chain alkyl, alkenyl or alkynyl group with up to 3 carbon atoms, and
R¹⁵ means a hydrogen atom
or
R¹⁴ and R¹⁵ together mean a methylene group that is optionally halogenated one or more times;
R^{15'} and R¹⁶, independently of one another, mean a hydrogen atom, a halogen atom, a group R¹⁸-O-, R¹⁹SO₂-O- or -R²², with R¹⁸, R¹⁹ and R²² in each case in the meaning that is indicated under R¹ and R²;
R¹⁷ and R^{17'} mean a hydrogen atom and a halogen atom; a hydrogen atom and a benzyloxy group; a hydrogen atom and a group R¹⁹SO₂-O-; a group R¹⁸ and a group -C(O)R²² or -O-C(O)R²², with R¹⁸, R¹⁹ and R²² in each case in the meaning that is indicated under R¹ and R², a group R¹⁸-O- and a group R¹⁸-; a group R¹⁸-O- and a group -O-C(O)R²², in all cases above with R¹⁸, R¹⁹ and R²² in each case in the meaning that is indicated under R¹ or R² and in the case of R¹⁸-O- with R¹⁸ additionally in the meaning of an aliphatic, straight-chain or branched-chain, saturated or unsaturated C₁-C₁₄ mono- or polycarboxylic acid or of an aromatic carboxylic acid or of an α- or β-amino acid; or
R¹⁷ and R^{17'} together mean a group =CR²³R²⁴, in which R²³ and R²⁴, independently of one another, represent a hydrogen atom or a halogen atom, or an oxygen atom;
and
one or more double bonds can be present in positions 6, 7; 7, 8; 9, 11; 11, 12; 14, 15 and also 15, 16 or 2 double bonds in the positions 6, 7 and 8, 9,
in the manufacture of a medicinal product for treating peri- and postmenopausal symptoms.

3. Use of gonatriene derivatives according to Claim 1 and also 18-nor-17β-estradiol in the manufacture of a medicinal product according to Claim 2.

4. Use of the gona-1,3,5(10)-triene derivatives of the general formula I'a according to Claim 2 in the manufacture of a medicament for treating peri- and postandropausal symptoms.

5. Use according to Claim 2 for prevention and treatment of hot flushes, sleep disturbances, irritability, mood swings, incontinence, vaginal atrophy and hormone deficiency induced emotional diseases.

6. Use of the gona-1,3,5(10)-triene derivatives of the general formula I'a according to Claim 2 in the manufacture of a medicament for prevention and treatment of diseases in the urogenital tract.

7. Use of the gona-1,3,5(10)-triene derivatives of the general formula I'a according to Claim 2 in the manufacture of a medicament for prevention and therapy of gastric and intestinal diseases.

8. Use according to Claim 7 for prevention and therapy of ulcers and haemorrhagic diatheses in the gastrointestinal tract.

9. Use according to Claim 7 for prevention and therapy of neoplasia.

10. Use of the gona-1,3,5(10)-triene derivatives of the general formula I'a according to Claim 2 in the manufacture of a medicament for the in vitro treatment of male infertility.

11. Use of the gona-1,3,5(10)-triene derivatives of the general formula I'a according to Claim 2 in the manufacture of a medicament for the in vivo treatment of male infertility.

12. Use of the gona-1,3,5(10)-triene derivatives of the general formula I'a according to Claim 2 in the manufacture of a medicament for the in vitro treatment of female infertility.

13. Use of the gona-1,3,5(10)-triene derivatives of the general formula I'a according to Claim 2 in the manufacture of a medicament for the in vivo treatment of female infertility.

14. Use according to Claim 2 for hormone replacement therapy (HRT).

15. Use of the gona-1,3,5(10)-triene derivatives of the general formula I'a according to Claim 2 in the manufacture of a medicament for therapy of hormone deficiency induced symptoms in surgically, medicamentally or otherwise induced ovarial dysfunction.

16. Use of the gona-1,3,5(10)-triene derivatives of the general formula I'a according to Claim 2 in the manufacture of a medicament for prophylaxis and therapy of a hormone deficiency induced loss of bone mass.

17. Use according to Claim 16 for prophylaxis and therapy of osteoporosis.

18. Use of gona-1,3,5(10)-triene derivatives according to Claim 1 in the manufacture of a medicament for prevention and therapy of cardiovascular diseases.

19. Use of gona-1,3,5(10)-triene derivatives according to Claim 1 in the manufacture of a medicament for prevention and treatment of vascular diseases.

20. Use of gona-1,3,5(10)-triene derivatives according to Claim 18 for prevention and treatment of atherosclerosis.

21. Use according to Claim 19 for prevention and treatment of neointimal hyperplasia.

22. Use of gona-1,3,5(10)-triene derivatives according to Claim 1 in the manufacture of a medicament for prevention and treatment of hormone deficiency induced neurodegenerative diseases.

23. Use of gona-1,3,5(10)-triene derivatives according to Claim 1 in the manufacture of a medicament for prevention and treatment of Alzheimer's disease and also hormone deficiency induced impairment of memory and learning capacity.

24. Use of gona-1,3,5(10)-triene derivatives according to Claim 1 in the manufacture of a medicament for treatment of inflammatory diseases and diseases of the immune system.

25. Use of gona-1,3,5(10)-triene derivatives according to Claim 1 in the manufacture of a medicament for prevention and treatment of benign prostate hyperplasia (BPH).

26. Pharmaceutical compositions comprising at least one compound according to Claim 1 and also a pharmaceutically compatible vehicle.

## Revendications

1. Dérivé de gonatriène selon la revendication 1, à savoir
11β-fluoro-gona-1,3,5(10)-triène-3,17-diol
11β-chloro-gona-1,3,5(10)-triène-3,17-diol
11β-méthyl-gona-1,3,5(10)-triène-3,17-diol
11β-éthyl-gona-1,3,5(10)-triène-3,17-diol
11β-phényl-gona-1,3,5(10)-triène-3,17-diol
7α-fluoro-gona-1,3,5(10)-triène-3,17-diol
7α-méthyl-gona-1,3,5(10)-triène-3,17β-diol
7α-phényl-gona-1,3,5(10)-triène-3,17β-diol
7α-méthyl-gona-1,3,5(10)-triène-3,17-diol
7β-éthyl-gona-1,3,5(10)-triène-3,17β-diol
7β-éthyl-gona-1,3,5(10)-triène-3,17α-diol
7β-éthyl-13α-H-gona-1,3,5(10)-triéne-3,17α-diol
7β-fluoro-gona-1,3,5(10)-triène-3,17-diol
7β-méthyl-gona-1,3,5(10)-triène-3,17β-diol
7β-phényl-gona-1,3,5(10)-triène-3,17β-diol
7β-méthyl-gona-1,3,5(10)-triène-3,17-diol
2-fluoro-gona-1,3,5(10)-triène-3,17β-diol
17α-(1-propynyl)-gona-1,3,5(10)-triène-3,17β-diol
11-méthylène-17a-(1-propynyl)-gona-1,3,5(10)-triène-3,17β-diol
11β-méthyl-17α-(1-propynyl)-gona-1,3,5(10)-triène-3,17β-diol
11β,17α-diméthyl-gona-1,3,5(10)-triène-3,17β-diol
11β,17β-diméthyl-gona-1,3,5(10)-triéne-3,17α-diol 18-nor-estriol
13α-H-18-nor-estradiol
11β-fluoro-gona-1,3,5(10)-triène-1,3,17-triol
11β-chloro-gona-1,3,5(10)-triène-1,3,17-triol
11β-méthyl-gona-1,3,5(10)-triène-1,3,17-triol
11β-éthyl-gona-1,3,5(10)-triène-1,3,17-txiol
11β-phényl-gona-1,3,5(10)-triène-1,3,17-triol
7α-fluoro-gona-1,3,5(10)-triène-1,3,17-triol
7α-méthyl-gona-1,3,5(10)-triène-1,3,17-triol
7α-phényl-gona-1,3,5(10)-triène-1,3,17-triol
7α-méthyl-gona-1,3,5(10)-triène-1,3,17-triol
7β-flucro-gona-1,3,5(10)-triène-1,3,17-triol
7β-méthyl-gona-1,3,5(10)-triène-1,3,17-triol
7β-phényl-gona-1,3,5(10)-triène-1,3,17-triol
7β-méthyl-gona-1,3,5(10)-triéne-1,3,17-triol
2-fluoro-gona-1,3,5(10)-triène-1,3,17-triol
17α-(1-propynyl)-gona-1,3,5(10)-triène-1,3,17-triol
11-méthylène-17α-(1-propynyl)-gona-1,3,5(10)-triène-1,3,17-triol
11β-méthyl-17α-(1-propynyl)-gona-1,3,5(10)-triène-1,3,17-triol
11β,17α-diméthyl-gona-1,3,5(10)-triène-1,3,17-triol
11β,17β-diméthyl-gona-1,3,5(10)-triène-1,3,17-triol.

2. Dérivés de gona-1,3,5(10)-triène de formule générale l'a 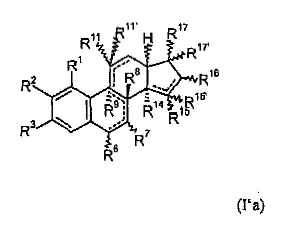 dans laquelle
R¹ représente un atome d'halogène, un radical R¹⁸- ou R¹⁸-O-, R¹⁶ représentant un atome d'hydrogène ou un radical hydrocarboné saturé ou insaturé, à chaîne droite ou ramifiée, ayant jusqu'à 6 atomes de carbone, ou un groupe trifluorométhyle ;
R² représente un atome d'halogène :
un radical R¹⁸- ou R¹⁸-O-, R¹⁸ ayant la signification indiquée sous R¹ ;
un groupe R¹⁹SO₂-O-, dans lequel R¹⁹ est un groupe R²⁰R²¹N, R²⁰ et R²¹ représentant, indépendamment l'un de l'autre, un atome d'hydrogène, un radical alkyle en C₁-C₅ également partiellement ou totalement fluoré ou substitué par 1-5 atomes d'halogène, groupes hydroxy ou groupes alcoxy en C₁-C₄, un groupe C(O)R²², dans lequel R²² représente un radical hydrocarboné à chaîne droite ou ramifiée ayant jusqu'à 12 atomes de carbone, qui peut en outre comporter jusqu'à trois doubles et/ou triples liaisons, un radical cycloalkyle en C₃-C₇, un radical aryle, le radical aryle incluant toujours également un radical hétéroaryle, un radical phényle, 1- ou 2-naphtyle, ou représente une combinaison de ces caractéristiques structurales ou, conjointement avec l'atome d'azote N, représente un radical polyméthylène-imino ayant de 4 à 6 atomes de carbone ou un radical morpholino ;
R³ représente un groupe R¹⁸-O-, R¹⁹SO₂-O- ou -O-C(O)R²², R¹⁸, R¹⁹ et R²² ayant chacun la signification indiquée sous R¹ ou R², R¹⁸ pouvant en outre représenter un radical aryle, hétéroaryle ou aralkyle, un radical aralkyle étant un radical contenant dans le cycle jusqu'à 14, de préférence 6 à 10 atomes de carbone et contenant de 1 à 8, de préférence de 1 à 4 atomes de carbone dans la chaîne alkyle, et un acide mono- ou polycarboxylique aliphatique, ramifié ou non ramifié, saturé ou insaturé, en C₁-C₁₄ ou un acide carboxylique aromatique ou un acide α- ou β-aminé ;
R⁶ et R⁷ représentent, indépendamment l'un de l'autre, un atome d'hydrogène, un atome d'halogène, un groupe R¹⁸-O-, R¹⁹SO₂-O- ou -R²², R¹⁸, R¹⁹ et R²² ayant chacun la signification indiquée sous R¹ ou R², un groupe alkyle à chaîne droite ou ramifiée, saturé ou insaturé, partiellement ou totalement halogéné, ayant jusqu'à 10 atomes de carbone ou un radical aryle, hétéroaryle ou aralkyle éventuellement substitué ;
R⁸ et R⁹ représentent, indépendamment l'un de l'autre un atome d'hydrogène, un radical hydrocarboné à chaîne droite ou ramifiée, saturé ou insaturé, éventuellement partiellement ou totalement halogéné, ayant jusqu'à 10 atomes de carbone, un groupe -X-R¹⁸, X étant un atome d'oxygène ou de soufre et R¹⁸ ayant la signification indiquée sous R¹, un atome d'halogène, un groupe cyano ou sulfocyano ;
R¹¹ représente un atome d'hydrogène, un atome d'halogène, un groupe R¹⁹SO₂-O- ou -R²², R¹⁸, R¹⁹ et R²² ayant chacun la signification indiquée sous R¹ ou R², un groupe -X-R¹⁸, X étant un atome d'oxygène ou de soufre et R¹⁸ ayant la signification indiquée sous R¹, un groupe nitro-oxy, un radical hydrocarboné à chaîne droite ou ramifiée, saturé ou insaturé, partiellement ou totalement halogéné, ayant jusqu'à 10 atomes de carbone, ou un radical aryle, hétéroaryle ou aralkyle éventuellement substitué, et
R^{11'} représente un atome d'hydrogène ou
R¹¹ et R^{11'} forment ensemble un groupe méthylène ;
R¹⁴ représente un atome d'hydrogène en position α ou un groupe alkyle, alcényle ou alcynyle à chaîne droite ou ramifiée, ayant jusqu'à 3 atomes de carbone et
R¹⁵ représente un atome d'hydrogène
ou
R¹⁴ et R¹⁵ forment ensemble un groupe méthylène éventuellement une ou deux fois halogéné ;
R^{15'} et R¹⁶ représentent, indépendamment l'un de l'autre, un atome d'hydrogène, un atome d'halogène, un groupe R¹⁸-O-, R¹⁹SO₂-O- ou -R²², R¹⁸, R¹⁹ et R²² ayant chacun la signification indiquée sous R¹ ou R² ;
R¹⁷ et R^{17'} représentent un atome d'hydrogène ou un atome d'halogène : un atome d'hydrogène et un groupe benzyloxy, un atome d'hydrogène et un groupe R¹⁹SO₂-O- ; un groupe R¹⁸ et un groupe -C(O)R²² ou -O-C(O)R²², R¹⁸, R¹⁹ et R²² ayant chacun la signification indiquée sous R¹ ou R² ; un groupe R¹⁸-O- ou un groupe R¹⁸- ; un groupe R¹⁸-O- et un groupe -O-C(O)R²², dans tous les cas précédents R¹⁸, R¹⁹ et R²² ayant chacun la signification indiquée sous R¹ ou R² et dans le cas de R¹⁸-O-, R¹⁸ pouvant représenter en outre un acide mono- ou polycarboxylique aliphatique en C₁-C₁₄ ramifié ou non ramifié, saturé ou insaturé, ou un acide carboxylique aromatique ou un acide α- ou β-aminé ; ou
R¹⁷ et R^{17'} représentent ensemble un groupe =CR²³R²⁴, R²³ et R²⁴ représentant indépendamment l'un de l'autre un atome d'hydrogène et un atome d'halogène, ou un atome d'oxygène ;
et
une ou plusieurs doubles liaisons peuvent être présentes dans les positions 6, 7 ; 7, 8 ; 9, 11 ; 11, 12 ; 14, 15 ainsi que 15, 16, ou 2 doubles liaisons peuvent être présentes dans les positions 6, 7 et 8, 9,
pour la fabrication d'un médicament destiné au traitement de troubles péri- et post-ménopausiques.

3. Utilisation de dérivés de gonatriène selon la revendication 1 ainsi que de 18-nor-17β-estradiol pour la fabrication d'un médicament selon la revendication 2.

4. Utilisation des dérivés de gona-1,3,5(10)-triène de formule générale I'a selon la revendication 2, pour la fabrication d'un médicament destiné au traitement de troubles péri- et post-andropausiques.

5. Utilisation selon la revendication 2, pour la prévention et le traitement de bouffées de chaleur, de troubles du sommeil, de l'irritabilité, de sautes d'humeur, de l'incontinence, de l'atrophie vaginale et de maladies psychiques dues à une déficience hormonale.

6. Utilisation des dérivés de gona-1,3,5(10)-triène de formule générale I'a selon la revendication 2, pour la fabrication d'un médicament destiné à la prévention et au traitement de maladies du tractus uro-génital.

7. Utilisation des dérivés de gona-1,3,5(10)-triène de formule générale l'a selon la revendication 2, pour la fabrication d'un médicament destiné à la prévention et la thérapie de maladies gastriques et intestinales.

8. Utilisation selon la revendication 7, pour la prévention et la thérapie d'ulcères et de diathèses hémorragiques dans le tractus gastro-intestinal.

9. Utilisation selon la revendication 7, pour la prévention et la thérapie de néoplasies.

10. Utilisation des dérivés de gona-1,3,5(10)-triène de formule générale l'a selon la revendication 2, pour la fabrication d'un médicament destiné au traitement in vitro de l'infertilité masculine.

11. Utilisation des dérivés de gona-1,3,5(10)-triène de formule générale l'a selon la revendication 2, pour la fabrication d'un médicament destiné au traitement in vivo de l'infertilité masculine.

12. Utilisation des dérivés de gona-1,3,5(10)-triène de formule générale I'a selon la revendication 2, pour la fabrication d'un médicament destiné au traitement in vitro de l'infertilité féminine.

13. Utilisation des dérivés de gona-1,3,5(10)-triéne de formule générale l'a selon la revendication 2, pour la fabrication d'un médicament destiné au traitement in vivo de l'infertilité féminine.

14. Utilisation selon la revendication 2, pour la thérapie hormonale substitutive (HRT).

15. Utilisation des dérivés de gona-1,3,5(10)-triène de formule générale l'a selon la revendication 2, pour la fabrication d'un médicament destiné à la thérapie de troubles dues à une déficience hormonale dans un dysfonctionnement ovarien d'origine chirurgicale, médicamenteuse ou autre.

16. Utilisation des dérivés de gona-1,3,5(10)-triène selon la revendication 2, pour la fabrication d'un médicament destiné à la prophylaxie et la thérapie d'une perte de masse osseuse due à une déficience hormonale.

17. Utilisation selon la revendication 16, pour la prophylaxie et la thérapie de l'ostéoporose.

18. Utilisation des dérivés de gona-1,3,5(10)-triène selon la revendication 1, pour la fabrication d'un médicament destiné à la prévention et la thérapie de maladies cardiovasculaires.

19. Utilisation des dérivés de gona-1,3,5(10)-triène selon la revendication 1, pour la fabrication d'un médicament destiné à la prévention et au traitement de maladies vasculaires.

20. Utilisation des dérivés de gona-1,3,5(10)-triène selon la revendication 18, pour la prévention et le traitement de l'athérosclérose.

21. Utilisation selon la revendication 19, pour la prévention et le traitement d'hyperplasies néo-intimales.

22. Utilisation des dérivés de gona-1,3,5(10)-triène selon la revendication 1, pour la fabrication d'un médicament destiné à la prévention et au traitement de maladies neurodégénératives dues à une déficience hormonale.

23. Utilisation des dérivés de gona-1,3,5(10)-triène selon la revendication 1, pour la fabrication d'un médicament destiné à la prévention et au traitement de la maladie d'Alzheimer ainsi que d'altération de la mémoire et de l'apprentissage, due à une déficience hormonale.

24. Utilisation des dérivés de gona-1,3,5(10)-triène selon la revendication 1, pour la fabrication d'un médicament destiné au traitement de maladies inflammatoires et de maladies du système immunitaire.

25. Utilisation des dérivés de gona-1,3,5(10)-triène selon la revendication 1, pour la fabrication d'un médicament destiné à la prévention et au traitement de l'hyperplasie bénigne de la prostate (BPH).

26. Compositions pharmaceutiques contenant au moins un composé selon la revendication 1, ainsi qu'un véhicule pharmaceutiquement acceptable.
